# EUROPEAN PATENT SPECIFICATION

(11) **EP 3 610 044 B1**
(45) Date of publication and mention of the grant of the patent: **05.01.2022**
(21) Application number: 18719227.3
(22) Date of filing: 11.04.2018
(51) Int. Cl.: C12Q 1/6895

(54) **DETECTION AND DELINEATION OF MICROORGANISMS USING THE ILV3 GENE**
NACHWEIS UND ABGRENZUNG VON MIKROORGANISMEN UNTER VERWENDUNG DES ILV3-GENS
DÉTECTION ET DÉTERMINATION DE MICRO-ORGANISMES À L'AIDE DU GÈNE ILV3

(30) Priority: 12.04.2017 GB 201705932; 25.07.2017 GB 201711949
(43) Date of publication of application: 19.02.2020
(73) Proprietor: Momentum Bioscience Limited, Cardiff, Wales CF3 0EF (GB)
(72) Inventor: ROGERS, Andrew, St. Mellons Cardiff CF3 0EF (GB)
(74) Representative: Boult Wade Tennant LLP
(86) International application number: PCT/GB2018/000065
(87) International publication number: WO 2018/189502

(56) References cited:
- WO-A2-2011/094356
- US-A1- 2003 180 953
- US-A1- 2011 081 348
- ANDALUZ ET AL: "Loss and fragmentation of chromosome 5 are major events linked to the adaptation of rad52-DELTADELTA strains of Candida albicans to sorbose", FUNGAL GENETICS AND BIOLOGY, vol. 44, no. 8, 2 June 2007 (2007-06-02), pages 789-798, XP022103305, SAN DIEGO, CA, US ISSN: 1087-1845, DOI: 10.1016/J.FGB.2007.01.005
- BURIK VAN J-A ET AL: "PANFUNGAL PCR ASSAY FOR DETECTION OF FUNGAL INFECTION IN HUMAN BLOOD SPECIMENS", JOURNAL OF CLINICAL MICROBIOLOGY, vol. 36, no. 5, 1 May 1998 (1998-05-01), pages 1169-1175, XP000961791, AMERICAN SOCIETY FOR MICROBIOLOGY, US ISSN: 0095-1137
- DATABASE AspGD [Online] Aspergillus Genome Database; 6 March 2012 (2012-03-06), "A. fumigatus ilv3/Afu2g14210 Summary", XP002782661, Database accession no. Afu2g14210
- DATABASE AspGD [Online] Aspergillus Genome Database; 24 June 2014 (2014-06-24), "C. albicans ILV3/C5_05410C Summary", XP002782662, Database accession no. C5_05410C
- DATABASE Nucleotide [Online] 17 August 2012 (2012-08-17), "Cryptococcus neoformans var. neoformans JEC21 dihydroxy-acid dehydratase, partial mRNA", XP002782663, retrieved from NCBI Database accession no. XM_572335
- JASON D. OLIVER ET AL: "The Aspergillus fumigatus Dihydroxyacid Dehydratase Ilv3A/IlvC Is Required for Full Virulence", PLOS ONE, vol. 7, no. 9, 18 September 2012 (2012-09-18), page e43559, XP055488042, DOI: 10.1371/journal.pone.0043559

## Description

### FIELD OF THE INVENTION

The invention provides a novel target in the context of detecting whether a fungus or yeast is present in a sample. This target, ILV3, encodes a dihydroxyacid dehydratase and is particularly useful in clinical diagnostic applications due to the lack of any sequence identity with the human genome. Primers and probes are provided which allow the presence or absence of *Candida, Aspergillus* and *Cryptococcus neoformans* to be determined in a sample. Once the presence of a fungus or yeast is determined, the identity of that species is usefully obtained, for example to direct therapy. As well as pan- *Candida* and pan-*Aspergillus* detection using a single primer/probe set there are also provided species-specific primer pairs that enable the species of *Candida* or *Aspergillus* to be determined in the sample. Melt curve analysis may be employed as a method of determining which species is present in the sample. The methods may be combined with methods that determine whether bacteria are present in the sample and which may categorise those bacteria as either Gram positive or Gram negative.

### BACKGROUND OF THE INVENTION

The US centre for disease control estimates that at least 30% of patients are prescribed antibiotics unnecessarily (Journal of the American Medical Association, May 2016). Antibiotic resistance is a pressing public health threat. Every year in the UK there are over 150,000 cases of sepsis resulting in 44,000 deaths. Many of these deaths are due to antibiotic resistant microbial infections. Culture-based identification of microbial infections takes around 5 days on average, during which time antibiotics are administered. It would be of great benefit if a more rapid test could be developed in order to detect a microbial infection in a sample.

Some molecular tests for identifying microbial DNA or RNA are known. For example, US7291724 and US7169555 describe oligonucleotides binding to ribosomal DNA that can be used in PCR reactions. Further amplification based methods for the detection of fungus/yeast are known. For example, see WO2002/27021, CN105018575, US6872523 and Van Burik et al., 1998. US2011/081348 discloses primers which hybridize to the ILV3 gene of *A. fumigatus.*

### DESCRIPTION OF THE INVENTION

The invention is defined in the appended claims.

Fungal infections (fungaemia) of the blood are highly dangerous. The inventors have investigated molecular targets that may be probed to rapidly (same day results) identify a fungal infection. The invention thus provides ILV3 as a novel target in the context of detecting whether a fungus or yeast is present in a sample. ILV3 encodes a dihydroxyacid dehydratase that catalyses the third step in the common pathway leading to biosynthesis of branched-chain amino acids. The inventors have discovered that this gene is present in the most clinically relevant fungal species and can be specifically targeted to permit detection and identification of a fungal infection. Moreover, this target is particularly useful in clinical diagnostic applications due to the lack of any sequence identity with the human genome. In more detail, Liu *et al.,* 2006 lists a number of candidate genes as potential targets for antifungal drug discovery. Of all the candidates listed, only ILV3 has a 0% identity with humans at the protein level. After conducting further analysis, the inventors have found that ILV3 also has no homology with humans or bacteria (of either Gram positive or Gram negative status) at the genetic level. This contrasts with the other candidate genes disclosed in Liu *et al.,* in which the inventors found genetic homology to humans. For example, ILV5 (2% identity with humans at the protein level) resulted in two positive nucleotide alignments (82% identity) with the human genome. Thus, candidate genes other than ILV3 (including ribosomal candidates, e.g. 18S, 28S or 5.8S) may result in the false positive detection and identification of a fungal infection in samples comprising human genetic material. Thus, in its broadest aspect, the invention provides for the use of ILV3 to identify a fungal infection in a sample.

It should be noted that throughout the specification the term "comprising" is intended to represent open-ended (i.e. including) language. However, for the avoidance of doubt, wherever the term "comprising" is used it is envisaged that the corresponding feature may be limited to that specified (i.e. consisting) as necessary.

Accession numbers and related information for the ILV3 gene sequence in the species of interest are provided in **Table A:**

| **Organism** | **Reference strain** | **Accession number** | **Gene ID** | **Gene symbol** | **Orientaion of gene** |
|---|---|---|---|---|---|
| *Candida albicans* | SC5314 | NC_032093.1 | 3636428 | ILV3 | Anti-sense |
| *Candida glabrata* | CBS138 | NC_005968.1 | 2886651 | CAGL0B03993g | Anti-sense |
| *Candida parapsilosis* | CDC317 | HE605203.1 | CPAR2_100130 (locus_tag) | CPAR2_100130 | Anti-sense |
| *Candida tropicalis* | MYA3404 | NW_003020040.1 | 8300008 | CTRG_06147 | Anti-sense |
| *Candida krusei* | SD108 | JQFK01000016.1 | JL09_g2096 | JL09_g2096 | Sense |
| | | | (locus tag) | | |
| *Candida dubliniensis* | CD36 | NC_012864.1 | 8048016 | CD36_55010 | Anti-sense |
| *Candida guilliermondii* | ATCC 6260 | NW_001809800.1 | 5129681 | PGUG_00520 | Anti-sense |
| *Candida auris* | 6684 | NW_017263971.1 | 28879407 | QG37_05711 | Anti-sense |
| *Aspergillus fumigatus* | Af293 | NC_007195.1 | 3512998 | AFUA_2G14210 | Sense |
| *Aspergillus niger* | CBS 513.88 | NT_166533.1 | 4989816 | ANI_1_1182164 | Anti-sense |
| *Aspergillus flavus* | NRRL3357 | NW_002477240.1 | 7922100 | AFLA_105610 | Anti-sense |
| *Cryptococcus neoformans* | JEC21 | NC_006693.1 | 3259119 | CNH01530 | Sense |

Through extensive characterisation and testing, the inventors have identified specific regions of the ILV3 gene that can be targeted. Firstly, the inventors have identified regions of the ILV3 gene in 8 clinically prevalent *Candida* species that can be commonly targeted using a single primer and/or probe set. A single primer and/or probe set specifically hybridises to ILV3 from these 8 species but does not cross-react with the ILV3 gene from non-*Candida* species. Such primer/probes may be referred to as "pan-*Candida*".

Secondly, the inventors have identified regions of the ILV3 gene in 3 clinically prevalent *Aspergillus* species that can be commonly targeted using a single primer and/or probe set. A single primer and/or probe set specifically hybridises to these 3 species but does not cross-react with the ILV3 gene from non-*Aspergillus* species. Such primer/probes may be referred to as "pan-*Aspergillus*".

Thirdly, the inventors have identified regions of the ILV3 gene in *Cryptococcus neoformans* that can be targeted using a primer and/or probe set. A primer and/or probe set specifically hybridises to this species but does not cross-react with the ILV3 gene from non-*Cryptococcus* species.

Collectively, the first, second and third category of target regions can be probed to determine whether there is a fungal infection in the sample. With appropriate discrimination of amplification products it can be determined which category of fungal infection is present.

Fourthly, the inventors have identified regions of the ILV3 gene that differ between 8 clinically prevalent *Candida* species that can thus each be separately targeted using suitably designed primer and/or probe sets. Each primer and/or probe set specifically hybridises to one *Candida* species but does not cross-react with the ILV3 gene from other *Candida* species (or non-*Candida* species).

Fifthly, the inventors have identified differing regions of the ILV3 gene in 3 clinically prevalent *Aspergillus* species that can thus each be separately targeted using suitably designed primer and/or probe sets. Each primer and/or probe set specifically hybridises to one *Aspergillus* species but does not cross-react with the ILV3 gene from other *Aspergillus* species (or non-*Aspergillus* species).

The fourth and fifth category of target regions can be probed to more specifically identify the nature of a fungal infection in the sample. With appropriate detection of amplification products the species responsible for the infection can be identified. This may facilitate treatment.

Specific primer and probe sequences are provided that target the respective regions and which also form an aspect of the present invention.

**Table B** below identifies the various ILV3 target regions, identified with reference to the sequences provided in Table A. Table B also provides specific primer and probe sequences of the invention that target the respective regions, together with the SEQ ID NO used in the sequence listing for each sequence:

| **Name** | **SEQ ID.** | **Forward primer sequence ⁽ⁱ⁾** | **Forward primer location ⁽ⁱⁱ⁾** | **SEQ ID.** | **Reverse primer sequence ⁽ⁱ⁾** | **Reverese primer location ⁽ⁱⁱ⁾** | **SEQ ID.** | **Probe sequence ⁽ⁱ⁾** | **Probe location ⁽ⁱⁱ⁾** |
|---|---|---|---|---|---|---|---|---|---|
| **Pan-Candid a (ILV3)** | 1 | GAAGGYCC AAARGGTG GWCC | CA: c1169825-1169806 CGI: c393798-393779 CT: c405996-405978 CDu: c1218036-1218017 CGu: c909809-909790 CP: c24006-23987 CK: 61666-61685 CAu: c32324-32305 | 2 | GAWCCACC MGARAATC TRCCRTC | CA: 1169707-1169729 CGI: 393680-393702 CT: 405878-405900 CDu: 1217918-1217940 CGu: 909691-909713 CP: 23888-23910 CK: c61784-61762 CAu: 32206-32228 | 3 | GAWGGY TTCAACAT TTCYGGC ATACC | CA: 1169779-1169804 CGI: 393752-393777 CT: 405950-405975 CDu: 1217990-1218015 CGu: 909763-909788 CP: 23960-23985 CK: c61712-61687 CAu: 32278-32303 |
| ***Candid a albican* s - melt (ILV3)** | 4 | TCCCTTGTT GGCCGATTT | c1170234-1170217 | 5 | TACCGTCGA TAATGCCTT CTTT | 1170119-1170140 | 116 | CGGCGGT ACACCTG CTGTTAT GAA | c1170174-1170151 |
| | 6 | AATCTTGCA GAGGGTGT CTTAG | c1171213-1171192 | 7 | GGGTCGCCT GTGACAATA G | 1171120-1171138 | 117 | TGCTTGA TTGTGGA TGTAGCA AATGTCC | 1171165-1171192 |
| ***Candid a dublinie nsis** -* **melt (ILV3)** | 8 | CAGTAAATA GGGCTGGC TTGA | c1219181-1219161 | 9 | CCTTCTGTA CCGTTGGTG ATAC | 1219103-1219124 | 118 | AATTGCA TGGCTTTC AAGCCAG CC | 1219148-1219171 |
| | 10 | TGCCTCGTC GTTTGACAT TA | c1218505-1218486 | 11 | GTACTTACC GGATGGCTT GAA | 1218409-1218429 | 119 | ATTAGCC GACTTCA AGCCATC CGG | c1218439-1218416 |
| | 12 | CATTGCCAC TGGTGGTTC TA | c1218553-1218534 | 13 | GATGGCTTG AAGTCGGCT AATA | 1218419-1218440 | 120 | TGCCTCG TCGTTTG ACATTACC ATCA | c1218505-1218480 |
| ***Candid a tropical is*** - **melt (ILV3)** | 14 | CCAGGTTCT GCTGTTGGT AA | c406146-406127 | 15 | CCAAAGCA GTGATGAA GGAATG | 406047-406068 | 121 | AGGTACT TACTTCAA GGGTAAA GCTAGAG T | c406111-406082 |
| | 16 | GTTCTGCTG TTGGTAAAA TCACT | c406142-406120 | 17 | CAAAGCAG TGATGAAG GAATGT | 406048-406069 | 121 | AGGTACT TACTTCAA GGGTAAA GCTAGAG T | c406111-406082 |
| ***Candid a parapsil osis -* melt (ILV3)** | 18 | GCCATGGG AAGACACA ATAGA | c24951-24931 | 19 | TCTTGTCGG CAATAGCTG GATTA | 24843-24865 | 122 | TACTATCT TGCCAGG GTCTCCC ACA | c24907-24883 |
| | 20 | GTGGTGGA AGCAAGAG TAA | c24881-24863 | 21 | GCTTCTCTT CCAAAGTG ATTTG | 24774-24795 | 123 | TCCTGCTA TTGCTGA CAAGATT GA | c24862-24839 |
| ***Candid a glabrat a* - melt (ILV3)** | 22 | TTCAAGCCA TCTGGTAAG TATGT | c394191-394169 | 23 | CAGTTAAG GCGTCACCG TATAA | 394080-394101 | 124 | AGGTCGG TGGTACT CAAAGTG TCA | c394151-394128 |
| | 24 | TAAGGCCG AATGGAAA CCTC | c393535-393516 | 25 | ACAACCTTG GGAGGCAT TAG | 393445-393464 | 125 | TGCTAAG TTAGTCTC TAATGCC TCCC | c393478-393453 |
| | 26 | GCCTCTCAG GCTATGTTG TATG | c395076-395055 | 27 | ACCAGACCA CCAACAAG AAC | 394987-395006 | 126 | TCAGCAA AGCGCAA GTTGGTG TTG | c395030-395007 |
| | 28 | GACGGTATC TCGATGGGT ACTA | c394884-394863 | 29 | ATCGTAGTG TTGTGCCAT CAT | 394783-394803 | 127 | AGAGAAA TTATCGC AGACTCT TTCGAGA CT | c394836-394807 |
| ***Candid a krusei* - melt (ILV3)** | 30 | ATGGGTTAC GGCTTAGGT AAAG | 61723-61744 | 31 | TCTGGAACA ATATGGCCG ATTA | c61817-61796 | 128 | ATTCTCTG GCGGTTC TCACGGT TT | 61770-61793 |
| | 32 | GGTGGTAT GTACACTGC CAATA | 60940-60961 | 33 | CCGAAACTG CTGGAGAT GAT | c61030-61011 | 129 | ACCCAAG ACTTCTG AGGCTGA AGC | c60990-60967 |
| | 34 | TGGTTTCAA GGACGAGG ATTT | 60420-60440 | 35 | CAGAATCTG CACATGCCT TATTT | c60535-60513 | 130 | TGGTTCTT GCTGGTG GTCTGGA AA | 60462-60485 |
| | 36 | GCGGTTCTC ACGGTTTCT TA | 61778-61797 | 37 | GGCAAGTTC TTCTTCGGA TACA | c61923-61902 | 131 | AGAAGCT TTCGAAG GCGGTCC AAT | 61815-61838 |
| | 38 | GGAAGAGG CCAGAGTTG AAATA | 61940-61961 | 39 | CCTTTGGAG GCATCAGA GAC | c62015-61996 | 132 | CCTCTCTT ATATTTCA ACTCTGG CCT | c61970-61945 |
| ***Candid a guillier mondii* - melt (ILV3)** | 40 | GTTCACGAC GGAGATGA GATTG | c909629-909608 | 41 | GGAGACCA CAGCTTCTT TCTTT | 909529-909550 | 133 | TGCTTGTC TCGGAAG AAATTCTC GCT | c909577-909552 |
| | 42 | TCGATCGTG ACCCAGGAT AA | c911117-911098 | 43 | GACCACCAT ACACTTCCA ACTC | 911002-911023 | 134 | TGCTTCG CAAGCCA TGTTGTAT GC | c911088-911065 |
| | 44 | GTGACCCA GGATAAGT CTCAAG | c911111-911090 | 45 | GGTTTCCTG ACCACCATA CA | 910994-911013 | 134 | TGCTTCG CAAGCCA TGTTGTAT GC | c911088-911065 |
| | 46 | CAAGGCTG GATTGAAA GGAATG | c910941-910920 | 47 | ACATCTGGG CCATCACTA AAG | 910799-910819 | 135 | ACCGTTG GTGATAC CATCAGA AACTCC | 910878-910904 |
| ***Candid a auris -* melt (ILV3)** | 48 | CTCCTCTGT AGGCGTTG AAATTA | c32790-32768 | 49 | CAAGTCAGC CATCACGTA CTTA | 32682-32703 | 136 | TCTGACA GCACTCC ATTGTTG GCT | c32744-32721 |
| | 50 | CACCGGTAA GGAAGGAA CATAC | c32451-32430 | 51 | CCAGCCTGT AAAGCAGT GATAA | 32368-32389 | 137 | AAAGGCT AGAGTGT TTGACGC CGA | c32421-32398 |
| | 52 | GCCAAGAT GTTGTTGGA AGAAG | c32654-32633 | 53 | GGCATTTGC TCAAGTTCT CTTT | 32567-32588 | 138 | TGCTTGA TGGTGAC CAGATGA CTGT | c32629-32605 |
| | 54 | AACATGCCT GGTGTGCTT AT | c33278-33259 | 55 | GGCATAAT GGTACCACC GTAAA | 33202-33223 | 139 | AAGGTCT GTTGTGT CTACCCAT GGC | 33233-33257 |
| | 56 | ACTGGTAA GACACTCAA AGAGAAC | c32603-32580 | 57 | GGTTTCAAT GGGTTGGA CAAAG | 32512-32533 | 140 | ATATCCT GGCCCTC AGGCAAG C | 32542-32563 |
| | 58 | GAGGAGGA CTTTATCAC TGCTTTA | c32399-32376 | 59 | TCTGATAAC ACACACGGT CTTT | 32328-32349 | 141 | TCGCCCTT CTTGATTT CTCCAGC C | 32350-32373 |
| | 60 | ATTGAACAA GCACTCCTC GAT | c33648-33628 | 61 | CCTTAAACC CAGTAGCGT ACAA | 33566-33587 | 142 | TGTCACC GAGGATG TGTCGCA G | c33627-33606 |
| | 62 | GCACTTCTA ACAGACGG AAGAT | c32240-32219 | 63 | TGGGACAA TGTGACCAA TCAA | 32175-32195 | 143 | TTTCTGGT GGATCCC ACGGTTT C | c32218-32196 |
| | 64 | GGTAAAGC CATCAGACA CTTGA | c32930-32909 | 65 | ACCTCCAGT GGCAATGA TATAAG | 32826-32848 | 144 | CCCGTGA CATCATG ACCAAGA | c32890-32864 |
| | | | | | | | | AATCGT | |
| | 66 | CATGGTTTA CGGTGGTA CCATTA | c33228-33206 | 67 | CCGTATGAT TGGAAAGC AGAGA | 33130-33151 | 145 | TGGCCAT GGGTAGA CACAACA GAC | c33259-33236 |
| | 68 | CAACAGAAT TGACTTGCT CGTG | c32106-32085 | 69 | CGCAAAGT ACCTCTCTT GTATCT | 32011-32033 | 146 | AAGAAAG AAGTGGC TGCCTCCT GA | c32061-32038 |
| **Pan-Aspergil lus V1 (ILV3)** | 70 | SCAGGGTG CTTCSCA | AFu: 3721583-3721597 AN: c541018-541004 AFI: c382612-382598 | 71 | TSGCRTCGT ACCACTG | AFu: c3721887-3721872 AN: 540714-540729 AFI: 382308-382323 | 72 | CAGTATG GGTACAA AGGGWA TGMGA | AFu: 3721790-3721814 AN: c540811-540788 AFI: c382405-382381 |
| **Pan-Aspergil lus V2 (ILV3)** | 73 | GGTACSAA GGGWATGC GATA | AFu: 3721797-3721816 AN: c540804-540785 AFI: c382398-382379 | 74 | CTGATGTTC GCRTCRTAC CA | AFu: c3721875-3721894 AN: 540707-540726 AFI: 382301-382320 | 75 | TCYATCG ARACCGT YATGGGT GG | AFu: 3721848-3721870 AN: c540753-540731 AFu: c382347-382325 |
| ***Aspergi llus fumigat* us (ILV3)** | 76 | GCGAAGAA ACGGCTTTG AATAA | 3721531-3721552 | 77 | TTGTACCAG ACAGACGA AATACC | c3721653-3721675 | 78 | ACCTGCG CCTTGTTC ATATCCTC C | c3721651-3721628 |
| | 79 | GCCTCACAG AGGAGGAT ATGA | 3721618-3721638 | 80 | ATCCAGCAG GTGCATGTT AC | c3721706-3721681 | 81 | TTCGTCTG TCTGGTA CAACGGC AA | 3721658-3721681 |
| | 82 | AGGCCTCAC AGAGGAGG ATATGA | 3721616-3721638 | 80 | ATCCAGCAG GTGCATGTT AC | c3721706-3721681 | 81 | TTCGTCTG TCTGGTA CAACGGC AA | 3721658-3721681 |
| | 83 | GTACCAAG GGAATGCG ATACT | 3721798-3721818 | 84 | GATACTGAT GTTCGCGTC GTA | c3721898-3721878 | 85 | TCCATCG AAACCGT CATGGGT GG | 3721848-3721870 |
| ***Aspergi llus niger* (ILV3)** | 87 | ATTGCTGAC TCTGTCGGT ATC | c540219-540199 | 86 | CTGGTGTGC CACCGATAT TA | 540084-540103 | 88 | AGACACC GTTCCTG GCTGATT TGA | c540161-540138 |
| ***Aspergi llus flavus* (ILV3)** | 90 | CTTCCCAGG CCATGCTTT A | c382604-382586 | 89 | GCTATACCA AACGGAGG AGATAC | 382519-382541 | 91 | ACCGAGG AGGACAT GAACAAA GCT | c382572-382549 |
| | 90 | CTTCCCAGG CCATGCTTT A | c382604-382586 | 92 | CTATACCAA ACGGAGGA GATAC | 382520-382541 | 91 | ACCGAGG AGGACAT GAACAAA GCT | c382572-382549 |
| ***Cryptoc occus neofor mans* (ILV3)** | 93 | GTTCAAGAC GGCGATGTT ATTC | 702696-702717 | 94 | GCAACCCA GTTCTCCTT TCT | c702796-702777 | 95 | ATGCCGT TGCGAAC ACTTTGTC TG | 702724-702747 |
| | 96 | GGTATGCCT GAAATGCTC AAAC | 702499-702520 | 97 | CCATGAGA ACCTCCGCT AAAT | c702602-702582 | 98 | AGTTTGA TCATGGG CGCTGGT CTA | 702526-702549 |
| | 99 | ACACTTTGT CTGTGGAC GTATC | 702736-702757 | 100 | GTAACAGCT CGGGCGTA TTT | c702850-702831 | 101 | TCAAGGT CACTCAC GGAACAT TGCT | 702805-702829 |
| | 102 | CGTCGAAA GCGGTTCTA TCA | 702384-702403 | 103 | GGTTTGAGC ATTTCAGGC ATAC | c702521-702500 | 104 | TGTTGTCT TGAGGTA CCTTGGC CC | 702423-702446 |
| | 105 | TCGACCCAG TATGATGGT TTATG | 701384-701406 | 106 | TTTGGCCTT CTTGGAGGT ATC | c701499-701479 | 107 | CGGAGAG GTGCTCG ACATTGT GTC | 701435-701458 |

(i) All sequences shown are in the 5' to 3' orientation
(ii) Primer / probe location is based on the orientation of the gene (c=complement) In Table B of GB1705932.0, the forward and reverse primer sequences and locations for *Aspergillus niger* (ILV3) and *Aspergillus flavus* (ILV3) were incorrectly interchanged and thus recited as follows:

| | | | | | | | | | |
|---|---|---|---|---|---|---|---|---|---|
| ***Aspergi llus niger* (ILV3)** | 86 | CTGGTGTGC CACCGATAT TA | 540084-540103 | 87 | ATTGCTGAC TCTGTCGGT ATC | c540219-540199 | 88 | AGACACC GTTCCTG GCTGATT TGA | c540161-540138 |
| ***Aspergi llus flavus* (ILV3)** | 89 | GCTATACCA AACGGAGG AGATAC | 382519-382541 | 90 | CTTCCCAGG CCATGCTTT A | c382604-382586 | 91 | ACCGAGG AGGACAT GAACAAA GCT | c382572-382549 |
| | 92 | CTATACCAA ACGGAGGA GATAC | 382520-382541 | 90 | CTTCCCAGG CCATGCTTT A | c382604-382586 | 91 | ACCGAGG AGGACAT GAACAAA GCT | c382572-382549 |

The designation of the terms "forward" and "reverse" primers is relative to the orientation of the ILV3 gene which is fixed for each of nucleotide sequence accession numbers NT_166533.1 (*Aspergillus niger*) and NW_002477240.1 (*Aspergillus flavus*). Thus, the skilled person consulting nucleotide sequence accession numbers NT_166533.1 and NW_002477240.1 when seeking to understand the invention, would immediately and unambiguously realise that the "forward" primer sequence and location as recited in Table B of GB1705932.0 is, in fact, the reverse primer sequence and location and *vice versa* for *Aspergillus niger* (ILV3) and *Aspergillus flavus* (ILV3). This correction has been applied throughout the present application.

Thus, the invention provides primers and probes useful in fungal detection. As would be readily understood by the skilled person, primers and probes hybridise to particular subregions within the gene of interest (ILV3). While the primers are specified individually herein, it would be immediately appreciated, based in particular on the information provided in Table B which primers are preferably paired according to the invention, including when defined by reference to their target region. Using the information provided herein, in particular the new target and specific target sequences, primers and probes may be designed by one skilled in the art. Typically, primers are between 15 and 40, such as between 18 and 35, nucleotides in length. Probes are typically between 15 and 100, such as between 20 and 40, nucleotides in length. Some mismatches to the target sequences may be tolerated provided that specific hybridisation is achieved. Specific hybridisation is a term of art well understood by the skilled person to exclude hybridisation to non-target sequences. The skilled person is also aware of suitable reaction conditions used for performing nucleic acid amplification under which specific hybridisation must occur. Moreover, for primers and/or probes which hybridise to multiple target sequences there may be some degeneracy in specific positions. For example, a primer may include any pyrimidine nucleotide (t/u or c) at a given position or a mixture of primers containing at least two of these nucleotides may be adopted. Variants of the specific primers and probes described herein (e.g. by SEQ ID NO) are also envisaged. They may contain nucleotide additions, deletions and/or substitutions provided that specific hybridisation is still achieved. 1, 2, 3, 4, 5, 6 or 7 additions, deletions and/or substitutions may be tolerated in some circumstances. As explained further herein, primers and/or probes may be labelled according to the detection methodology employed. Typical labels are fluorescent molecules, which may be arranged as fluorophores and quenchers in some aspects.

Described herein is at least one primer pair for detecting a yeast/fungus infection in a sample comprising a forward and reverse primer hybridizing specifically to the ILV3 gene of the following *Candida* species:
i. *Candida albicans*
ii. *Candida dubliniensis*
iii. *Candida tropicalis*
iv. *Candida parapsilosis*
v. *Candida glabrata*
vi. *Candida krusei*
*vii. Candida guilliermondii*
viii. *Candida auris*

By "hybridising specifically", or equivalent language, is meant that the primers hybridise to ILV3 from these 8 species but do not hybridise (or cross-react) with the ILV3 gene from non-*Candida* species. Thus, an amplification product will only be generated if a *Candida* species (from those 8 species) is present in the sample.

According to some embodiments, the forward primer of a primer pair hybridises to at least 3, 4, 5, 6, 7 and preferably all of the following target sequences:
i. positions c1169825-1169806 of nucleotide sequence accession number NC_032093.1 (*Candida albicans*)
ii. positions c393798-393779 of nucleotide sequence accession number NC_005968.1 (*Candida glabrata*)
iii. positions c405996-405978 of nucleotide sequence accession number NW_003020040.1 (*Candida tropicalis*)
iv. positions c1218036-1218017 of nucleotide sequence accession number NC_012864.1 (*Candida dubliniensis*)
v. positions c909809-909790 of nucleotide sequence accession number NW_001809800.1 (*Candida guilliermondii*)
vi. positions c24006-23987 of nucleotide sequence accession number HE605203.1 (*Candida parapsilosis*)
vii. positions 61666-61685 of nucleotide sequence accession number JQFK01000016.1 (*Candida krusei*)
viii. positions c32324-32305 of nucleotide sequence accession number NW_017263971.1 (*Candida auris*).

According to some embodiments, the reverse primer of a primer pair hybridises to at least 3, 4, 5, 6, 7 and preferably all of the following target sequences:
i. positions 1169707-1169729 of nucleotide sequence accession number NC_032093.1 (*Candida albicans*)
ii. positions 393680-393702 of nucleotide sequence accession number NC_005968.1 (*Candida glabrata*)
iii. positions 405878-405900 of nucleotide sequence accession number NW_003020040.1 (*Candida tropicalis*)
iv. positions 1217918-1217940 of nucleotide sequence accession number NC_012864.1 (*Candida dubliniensis*)
v. positions 909691-909713 of nucleotide sequence accession number NW_001809800.1 (*Candida guilliermondii*)
vi. positions 23888-23910 of nucleotide sequence accession number HE605203.1 (*Candida parapsilosis*)
vii. positions c61784-61762 of nucleotide sequence accession number JQFK01000016.1 (*Candida krusei*)
viii. positions 32206-32228 of nucleotide sequence accession number NW_017263971.1 (*Candida auris*).

Thus, primer pairs may be generated from these particular target regions to permit pan-*Candida* detection. This can be achieved with a single primer pair in some embodiments.

In specific embodiments, the forward and reverse primer hybridizing specifically to the ILV3 gene of at least 3, 4, 5, 6, 7 and preferably all of the *Candida* species
i. *Candida albicans*
ii. *Candida dubliniensis*
iii. *Candida tropicalis*
iv. *Candida parapsilosis*
v. *Candida glabrata*
vi. *Candida krusei*
vii. *Candida guilliermondii*
viii. *Candida auris*
comprises, consists essentially of or consists of the nucleotide sequence of SEQ ID NO: 1 and SEQ ID NO: 2 respectively. This is a pan-Candida primer pair.

Described herein is a forward and reverse primer hybridizing specifically to the ILV3 gene of the following *Aspergillus* species
i. *Aspergillus fumigatus*
ii. *Aspergillus niger*
iii. *Aspergillus flavus*

By "hybridising specifically", or equivalent language, is meant that the primers hybridise to ILV3 from these 3 species but do not hybridise (or cross-react) with the ILV3 gene from non-*Aspergillus* species. Thus, an amplification product will only be generated if an *Aspergillus* species (from those 3 species) is present in the sample.

According to some embodiments, the forward primer of a primer pair hybridises to at least 2, and preferably all 3, of the following target sequences:
i. positions 3721583-3721597 of nucleotide sequence accession number NC_007195.1 (*Aspergillus fumigatus*)
ii. positions c541018-541004 of nucleotide sequence accession number NT_166533.1 (*Aspergillus niger*)
iii. positions c382612-382598 of nucleotide sequence accession number NW_002477240.1 (*Aspergillus flavus*)
or wherein the forward primer hybridises to at least 2, and preferably all 3, of the following target sequences:
i. positions 3721797-3721816 of nucleotide sequence accession number NC_007195.1 (*Aspergillus fumigatus*)
ii. positions c540804-540785 of nucleotide sequence accession number NT_166533.1 (*Aspergillus niger*)
iii. positions c382398-382379 of nucleotide sequence accession number NW_002477240.1 (*Aspergillus flavus*)

According to some embodiments, the reverse primer of a primer pair hybridises to at least 2, and preferably all 3, of the following target sequences:
i. positions c3721887-3721872 of nucleotide sequence accession number NC_007195.1 (*Aspergillus fumigatus*)
ii. positions 540714-540729 of nucleotide sequence accession number NT_166533.1 (*Aspergillus niger*)
iii. positions 382308-382323 of nucleotide sequence accession number NW_002477240.1 (*Aspergillus flavus*)
or wherein the reverse primer hybridises to at least 2, and preferably all 3, of the following target sequences:
i. positions c3721875-3721894 of nucleotide sequence accession number NC_007195.1 (*Aspergillus fumigatus*)
ii. positions 540707-540726 of nucleotide sequence accession number NT_166533.1 (*Aspergillus niger*)
iii. positions 382301-382320 of nucleotide sequence accession number NW_002477240.1 (*Aspergillus flavus*)

Thus, primer pairs may be generated from these particular target regions to permit pan-*Aspergillus* detection. This can be achieved with a single primer pair in some embodiments.

In specific embodiments the forward and reverse primer hybridizing specifically to the ILV3 gene of the following *Aspergillus* species
i. *Aspergillus fumigatus*
ii. *Aspergillus niger*
iii. *Aspergillus flavus*
comprises, consists essentially of or consists of the nucleotide sequence of SEQ ID NO: 70 and 71 or SEQ ID NO: 73 and 74 respectively. Thus, SEQ ID NO: 70 and 71 form a preferred primer pair. SEQ ID NO: 73 and 74 form a second primer pair. These are pan-*Aspergillus* primer pairs.

Described herein is a forward and reverse primer hybridizing specifically to the ILV3 gene of *Candida albicans.*

By "hybridising specifically", or equivalent language, is meant that the primers hybridise to ILV3 from this species but do not hybridise (or cross-react) with the ILV3 gene from non-*Candida albicans* species. Thus, an amplification product will only be generated if *Candida albicans* is present in the sample and will not be generated if one of the other 7 *Candida* species is present in the sample (or if a non-Candida species is present).

According to some embodiments, the forward primer of a primer pair hybridizing specifically to the ILV3 gene of *Candida albicans* hybridises to one of the following target sequences from nucleotide sequence accession number NC_032093.1:
i. positions c1170234-1170217
ii. positions c1171213-1171192

According to some embodiments, the reverse primer of a primer pair hybridizing specifically to the ILV3 gene of *Candida albicans* hybridises to one of the following target sequences from nucleotide sequence accession number NC_032093.1:
i. positions 1170119-1170140
ii. positions 1171120-1171138

In specific embodiments the forward and reverse primer hybridizing specifically to the ILV3 gene of *Candida albicans* comprises, consists essentially of or consists of the nucleotide sequence of SEQ ID NO: 4 and 5 or 6 and 7 respectively. Thus, SEQ ID NO: 4 and 5 form a first primer pair. SEQ ID NO: 6 and 7 form a second primer pair.

Described herein is a forward and reverse primer hybridizing specifically to the ILV3 gene of *Candida dubliniensis.*

By "hybridising specifically", or equivalent language, is meant that the primers hybridise to ILV3 from this species but do not hybridise (or cross-react) with the ILV3 gene from non-*Candida dubliniensis* species. Thus, an amplification product will only be generated if *Candida dubliniensis* is present in the sample and will not be generated if one of the other 7 *Candida* species is present in the sample (or if a non-*Candida* species is present).

According to some embodiments, the forward primer of a primer pair hybridizing specifically to the ILV3 gene of *Candida dubliniensis* hybridises to one of the following target sequences from nucleotide sequence accession number NC_012864.1:
i. positions c1219181-1219161
ii. positions c1218505-1218486
iii. positions c1218553-1218534

According to some embodiments, the reverse primer of a primer pair hybridizing specifically to the ILV3 gene of *Candida dubliniensis* hybridises to one of the following target sequences from nucleotide sequence accession number NC_012864.1:
i. positions 1219103-1219124
ii. positions 1218409-1218429
iii. positions 1218419-1218440

In specific embodiments the forward and reverse primer hybridizing specifically to the ILV3 gene of *Candida dubliniensis* comprises, consists essentially of or consists of the nucleotide sequence of SEQ ID NO: 8 and 9, SEQ ID NO: 10 and 11 or SEQ ID NO: 12 and 13 respectively. Thus, SEQ ID NO: 8 and 9 form a first primer pair. SEQ ID NO: 10 and 11 form a second primer pair and so on.

Described herein is a forward and reverse primer hybridizing specifically to the ILV3 gene of *Candida tropicalis.*

By "hybridising specifically", or equivalent language, is meant that the primers hybridise to ILV3 from this species but do not hybridise (or cross-react) with the ILV3 gene from non-*Candida tropicalis* species. Thus, an amplification product will only be generated if *Candida tropicalis* is present in the sample and will not be generated if one of the other 7 *Candida* species is present in the sample (or if a non-*Candida* species is present).

According to some embodiments, the forward primer of a primer pair hybridizing specifically to the ILV3 gene of *Candida tropicalis* hybridises to one of the following target sequences from nucleotide sequence accession number NW_003020040.1:
i. positions c406146-406127
ii. positions c406142-406120

According to some embodiments, the reverse primer of a primer pair hybridizing specifically to the ILV3 gene of *Candida tropicalis* hybridises to one of the following target sequences from nucleotide sequence accession number NW_003020040.1:
i. positions 406047-406068
ii. positions 406048-406069

In specific embodiments the forward and reverse primer hybridizing specifically to the ILV3 gene of *Candida tropicalis* comprises, consists essentially of or consists of the nucleotide sequence of SEQ ID NO: 14 and 15 or SEQ ID NO: 16 and 17 respectively. Thus, SEQ ID NO: 14 and 15 form a first primer pair. SEQ ID NO: 16 and 17 form a second primer pair.

Described herein is a forward and reverse primer hybridizing specifically to the ILV3 gene of *Candida parapsilosis.*

By "hybridising specifically", or equivalent language, is meant that the primers hybridise to ILV3 from this species but do not hybridise (or cross-react) with the ILV3 gene from non-*Candida parapsilosis* species. Thus, an amplification product will only be generated if *Candida parapsilosis* is present in the sample and will not be generated if one of the other *7 Candida* species is present in the sample (or if a non-*Candida* species is present).

According to some embodiments, the forward primer of a primer pair hybridizing specifically to the ILV3 gene of *Candida parapsilosis* hybridises to one of the following target sequences from nucleotide sequence accession number HE605203.1:
i. positions c24951-24931
ii. positions c24881-24863

According to some embodiments, the reverse primer of a primer pair hybridizing specifically to the ILV3 gene of *Candida parapsilosis* hybridises to one of the following target sequences from nucleotide sequence accession number HE605203.1:
i. positions 24843-24865
ii. positions 24774-24795

In specific embodiments the forward and reverse primer hybridizing specifically to the ILV3 gene of *Candida parapsilosis* comprises, consists essentially of or consists of the nucleotide sequence of SEQ ID NO: 18 and 19 or SEQ ID NO: 20 and 21 respectively. Thus, SEQ ID NO: 18 and 19 form a first primer pair. SEQ ID NO: 20 and 21 form a second primer pair.

Described herein is a forward and reverse primer hybridizing specifically to the ILV3 gene of *Candida glabrata.*

By "hybridising specifically", or equivalent language, is meant that the primers hybridise to ILV3 from this species but do not hybridise (or cross-react) with the ILV3 gene from non-*Candida glabrata* species. Thus, an amplification product will only be generated if *Candida glabrata* is present in the sample and will not be generated if one of the other 7 *Candida* species is present in the sample (or if a non-*Candida* species is present).

According to some embodiments, the forward primer of a primer pair hybridizing specifically to the ILV3 gene of *Candida glabrata* hybridises to one of the following target sequences from nucleotide sequence accession number NC_005968.1:
i. positions c394191-394169
ii. positions c393535-393516
iii. positions c395076-395055
iv. positions c394884-394863

According to some embodiments, the reverse primer of a primer pair hybridizing specifically to the ILV3 gene of *Candida glabrata* hybridises to one of the following target sequences from nucleotide sequence accession number NC_005968.1:
i. positions 394080-394101
ii. positions 393445-393464
iii. positions 394987-395006
iv. positions 394783-394803

In specific embodiments the forward and reverse primer hybridizing specifically to the ILV3 gene of *Candida glabrata* comprises, consists essentially of or consists of the nucleotide sequence of SEQ ID NO: 22 and 23, SEQ ID NO: 24 and 25, SEQ ID NO: 26 and 27 or SEQ ID NO: 28 and 29 respectively. Thus, SEQ ID NO: 22 and 23 form a first primer pair. SEQ ID NO: 24 and 25 form a second primer pair and so on.

Described herein is a forward and reverse primer hybridizing specifically to the ILV3 gene of *Candida krusei.*

By "hybridising specifically", or equivalent language, is meant that the primers hybridise to ILV3 from this species but do not hybridise (or cross-react) with the ILV3 gene from non-*Candida krusei* species. Thus, an amplification product will only be generated if *Candida krusei* is present in the sample and will not be generated if one of the other 7 *Candida* species is present in the sample (or if a non-Candida species is present).

According to some embodiments, the forward primer of a primer pair hybridizing specifically to the ILV3 gene of *Candida krusei* hybridises to one of the following target sequences from nucleotide sequence accession number JQFK01000016.1:
i. positions 61723-61744
ii. positions 60940-60961
iii. positions 60420-60440
iv. positions 61778-61797
v. positions 61940-61961

According to some embodiments, the reverse primer of a primer pair hybridizing specifically to the ILV3 gene of *Candida krusei* hybridises to one of the following target sequences from nucleotide sequence accession number JQFK01000016.1:
i. positions c61817-61796
ii. positions c61030-61011
iii. positions c60535-60513
iv. positions c61923-61902
v. positions c62015-61996

In specific embodiments the forward and reverse primer hybridizing specifically to the ILV3 gene of *Candida krusei* comprises, consists essentially of or consists of the nucleotide sequence of SEQ ID NO: 30 and 31, SEQ ID NO: 32 and 33, SEQ ID NO: 34 and 35, SEQ ID NO: 36 and 37 or SEQ ID NO: 38 and 39 respectively. Thus, SEQ ID NO: 30 and 31 form a first primer pair. SEQ ID NO: 32 and 33 form a second primer pair and so on.

Described herein is a forward and reverse primer hybridizing specifically to the ILV3 gene of *Candida guilliermondii.*

By "hybridising specifically", or equivalent language, is meant that the primers hybridise to ILV3 from this species but do not hybridise (or cross-react) with the ILV3 gene from non-*Candida guilliermondii* species. Thus, an amplification product will only be generated if *Candida guilliermondii* is present in the sample and will not be generated if one of the other *7 Candida* species is present in the sample (or if a non-*Candida* species is present).

According to some embodiments, the forward primer of a primer pair hybridizing specifically to the ILV3 gene of *Candida guilliermondii* hybridises to one of the following target sequences from nucleotide sequence accession number NW_001809800.1:
i. positions c909629-909608
ii. positions c911117-911098
iii. positions c911111-911090
iv. positions c910941-910920

According to some embodiments, the reverse primer of a primer pair hybridizing specifically to the ILV3 gene of *Candida guilliermondii* hybridises to one of the following target sequences from nucleotide sequence accession number NW_001809800.1:
i. positions 909529-909550
ii. positions 911002-911023
iii. positions 910994-911013
iv. positions 910799-910819

In specific embodiments the forward and reverse primer hybridizing specifically to the ILV3 gene of *Candida guilliermondii* comprises, consists essentially of or consists of the nucleotide sequence of SEQ ID NO: 40 and 41, SEQ ID NO: 42 and 43, SEQ ID NO: 44 and 45 or SEQ ID NO: 46 and 47 respectively. Thus, SEQ ID NO: 40 and 41 form a first primer pair. SEQ ID NO: 42 and 43 form a second primer pair and so on.

Described herein is a forward and reverse primer hybridizing specifically to the ILV3 gene of *Candida auris.*

By "hybridising specifically", or equivalent language, is meant that the primers hybridise to ILV3 from this species but do not hybridise (or cross-react) with the ILV3 gene from non-*Candida auris* species. Thus, an amplification product will only be generated if *Candida auris* is present in the sample and will not be generated if one of the other 7 *Candida* species is present in the sample (or if a non-*Candida* species is present).

According to some embodiments, the forward primer of a primer pair hybridizing specifically to the ILV3 gene of *Candida auris* hybridises to one of the following target sequences from nucleotide sequence accession number NW_017263971.1:
i. positions c32790-32768
ii. positions c32451-32430
iii. positions c32654-32633
iv. positions c33278-33259
v. positions c32603-32580
vi. positions c32399-32376
vii. positions c33648-33628
viii. positions c32240-32219
ix. positions c32930-32909
x. positions c33228-33206
xi. positions c32106-32085

According to some embodiments, the reverse primer of a primer pair hybridizing specifically to the ILV3 gene of *Candida auris* hybridises to one of the following target sequences from nucleotide sequence accession number NW_017263971.1:
i. positions 32682-32703
ii. positions 32368-32389
iii. positions 32567-32588
iv. positions 33202-33223
v. positions 32512-32533
vi. positions 32328-32349
vii. positions 33566-33587
viii. positions 32175-32195
ix. positions 32826-32848
x. positions 33130-33151
xi. positions 32011-32033

In specific embodiments the forward and reverse primer hybridizing specifically to the ILV3 gene of *Candida auris* comprises, consists essentially of or consists of the nucleotide sequence of SEQ ID NO: 48 and 49, SEQ ID NO: 50 and 51, SEQ ID NO: 52 and 53, SEQ ID NO: 54 and 55, SEQ ID NO: 56 and 57, SEQ ID NO: 58 and 59, SEQ ID NO: 60 and 61, SEQ ID NO: 62 and 63, SEQ ID NO: 64 and 65, SEQ ID NO: 66 and 67 or SEQ ID NO: 68 and 69 respectively. Thus, SEQ ID NO: 48 and 49 form a first primer pair. SEQ ID NO: 50 and 51 form a second primer pair and so on.

Described herein is a forward and reverse primer hybridizing specifically to the ILV3 gene of *Aspergillus fumigatus.*

By "hybridising specifically", or equivalent language, is meant that the primers hybridise to ILV3 from this species but do not hybridise (or cross-react) with the ILV3 gene from non-*Aspergillus fumigatus* species. Thus, an amplification product will only be generated if *Aspergillus fumigatus* is present in the sample and will not be generated if one of the other 2 *Aspergillus* species is present in the sample (or if a non-*Aspergillus* species is present).

According to some embodiments, the forward primer of a primer pair hybridizing specifically to the ILV3 gene of *Aspergillus fumigatus* hybridises to one of the following target sequences from nucleotide sequence accession number NC_007195.1:
i. positions 3721531-3721552
ii. positions 3721618-3721638
iii. positions 3721616-3721638
iv. positions 3721798-3721818

According to some embodiments, the reverse primer of a primer pair hybridizing specifically to the ILV3 gene of *Aspergillus fumigatus* hybridises to one of the following target sequences from nucleotide sequence accession number NC_007195.1:
i. positions c3721653-3721675
ii. positions c3721706-3721681
iii. positions c3721898-3721878

In specific embodiments the forward and reverse primer hybridizing specifically to the ILV3 gene of *Aspergillus fumigatus* comprises, consists essentially of or consists of the nucleotide sequence of SEQ ID NO: 76 and 77, SEQ ID NO: 79 and 80, SEQ ID NO: 82 and 80 or SEQ ID NO: 83 and 84 respectively. Thus, SEQ ID NO: 76 and 77 form a first primer pair. SEQ ID NO: 79 and 80 form a second primer pair and so on.

Described herein is a forward and reverse primer hybridizing specifically to the ILV3 gene of *Aspergillus niger.*

By "hybridising specifically", or equivalent language, is meant that the primers hybridise to ILV3 from this species but do not hybridise (or cross-react) with the ILV3 gene from non-*Aspergillus niger* species. Thus, an amplification product will only be generated if *Aspergillus niger* is present in the sample and will not be generated if one of the other 2 *Aspergillus* species is present in the sample (or if a non-*Aspergillus* species is present). According to some embodiments, the forward primer of a primer pair hybridizing specifically to the ILV3 gene of *Aspergillus niger* hybridises to one of the following target sequences from nucleotide sequence accession number NT_166533.1:
i. positions c540219-540199

According to some embodiments, the reverse primer of a primer pair hybridizing specifically to the ILV3 gene of *Aspergillus niger* hybridises to one of the following target sequences from nucleotide sequence accession number NT_166533.1:
ii. positions 540084-540103

In specific embodiments the forward and reverse primer hybridizing specifically to the ILV3 gene of *Aspergillus niger* comprises, consists essentially of or consists of the nucleotide sequence of SEQ ID NO: 87 and 86.

Described herein is a forward and reverse primer hybridizing specifically to the ILV3 gene of *Aspergillus flavus.*

By "hybridising specifically", or equivalent language, is meant that the primers hybridise to ILV3 from this species but do not hybridise (or cross-react) with the ILV3 gene from non-*Aspergillus flavus* species. Thus, an amplification product will only be generated if *Aspergillus flavus* is present in the sample and will not be generated if one of the other 2 *Aspergillus* species is present in the sample (or if a non-*Aspergillus* species is present).

According to some embodiments, the forward primer of a primer pair hybridizing specifically to the ILV3 gene of *Aspergillus flavus* hybridises to one of the following target sequences from nucleotide sequence accession number NW_002477240.1:
i. positions c382604-382586

According to some embodiments, the reverse primer of a primer pair hybridizing specifically to the ILV3 gene of *Aspergillus flavus* hybridises to one of the following target sequences from nucleotide sequence accession number NW_002477240.1:
i. positions 382519-382541
ii. positions 382520-382541

In specific embodiments the forward and reverse primer hybridizing specifically to the ILV3 gene of *Aspergillus flavus* comprises, consists essentially of or consists of the nucleotide sequence of SEQ ID NO: 90 and 89 or SEQ ID NO: 90 and 92 respectively. Thus, SEQ ID NO: 90 and 89 form a first primer pair. SEQ ID NO: 90 and 92 form a second primer pair.

Described herein is a forward and reverse primer hybridizing specifically to the ILV3 gene of *Cryptococcus neoformans.*

By "hybridising specifically", or equivalent language, is meant that the primers hybridise to ILV3 from this species but do not hybridise (or cross-react) with the ILV3 gene from non-*Cryptococcus neoformans* species. Thus, an amplification product will only be generated if *Cryptococcus neoformans* is present in the sample and will not be generated if a non-*Cryptococcus neoformans* species is present.

According to some embodiments, the forward primer of a primer pair hybridizing specifically to the ILV3 gene of *Cryptococcus neoformans* hybridises to one of the following target sequences from nucleotide sequence accession number NC_006693.1:
i. Positions 702696-702717
ii. Positions 702499-702520
iii. Positions 702736-702757
iv. Positions 702384-702403
v. Positions 701384-701406

According to some embodiments, the reverse primer of a primer pair hybridizing specifically to the ILV3 gene of *Cryptococcus neoformans* hybridises to one of the following target sequences from nucleotide sequence accession number NC_006693.1:
i. Positions c702796-702777
ii. Positions c702602-702582
iii. Positions c702850-702831
iv. Positions c702521-702500
v. Positions c701499-701479

In specific embodiments the forward and reverse primer hybridizing specifically to the ILV3 gene of *Cryptococcus neoformans* comprises, consists essentially of or consists of the nucleotide sequence of SEQ ID NO: 93 and 94, SEQ ID NO: 96 and 97, SEQ ID NO: 99 and 100, SEQ ID NO: 102 and 103 or SEQ ID NO: 105 and 106 respectively. Thus, SEQ ID NO: 93 and 94 form a first primer pair. SEQ ID NO: 95 and 97 form a second primer pair and so on.

The primer pairs, as explained herein, are preferably used in combination for example in multiplex reactions. Multiple primer pairs can be included in a single reaction mixture (a mastermix). Thus, in some embodiments, at least one primer in each primer pair is differentially labelled compared to the other primer pairs. This is one means by which amplification products can be distinguished. Examples of labelled primers that may be used in the present invention include AMPLIFLUOR primers and LUX primers. Thus primers may include modifications, labels and sequence extensions to incorporate the relevant detection technology. Such sequence modifications, labels and extensions are encompassed by the invention.

Many nucleic acid amplification protocols involve use of a probe. Variants of PCR permit detection in real time or at end-point using such probes. Examples include hydrolytic probes (e.g. TAQMAN probes) and hairpin probes (e.g. MOLECULAR BEACONS). Probes may also be attached to primers in some embodiments (e.g. SCORPION probes). Thus probes of the invention may include modifications, labels and sequence extensions to incorporate the relevant detection technology. Such sequence modifications, labels and extensions are encompassed by the invention. Preferably, the probes of the invention also target the ILV3 gene in genus or species specific fashion to complement the action of the primers. In relation to the probes of the invention "hybridising specifically" is defined in analogous fashion to the definitions provided for the corresponding primers. Again, Table B identifies preferred combinations of probes of the invention with primer pairs of the invention, including when defined by reference to their target sequence.

Described herein is at least one probe for detecting a yeast/fungus infection in a sample comprising a probe that hybridizes specifically to the ILV3 gene of the following *Candida* species
i. *Candida albicans*
ii. *Candida dubliniensis*
iii. *Candida tropicalis*
iv. *Candida parapsilosis*
v. *Candida glabrata*
vi. *Candida krusei*
*vii. Candida guilliermondii*
viii. *Candida auris*

According to some instances the at least one probe hybridises to at least 3, 4, 5, 6, 7 and preferably all of the following target sequences:
i. positions 1169779-1169804 of nucleotide sequence accession number NC_032093.1 (*Candida albicans*)
ii. positions 393752-393777 of nucleotide sequence accession number NC_005968.1 (*Candida glabrata*)
iii. positions 405950-405975 of nucleotide sequence accession number NW_003020040.1 (*Candida tropicalis*)
iv. positions 1217990-1218015 of nucleotide sequence accession number NC_012864.1 (*Candida dubliniensis*)
v. positions 909763-909788 of nucleotide sequence accession number NW_001809800.1 (*Candida guilliermondii*)
vi. positions 23960-23985 of nucleotide sequence accession number HE605203.1 (*Candida parapsilosis*)
vii. positions c61712-61687 of nucleotide sequence accession number JQFK01000016.1 (*Candida krusei*)
viii. positions 32278-32303 of nucleotide sequence accession number NW_017263971.1 (*Candida auris*)*.*

In specific embodiments, the probe that hybridizes specifically to the ILV3 gene of the following *Candida* species
i. *Candida albicans*
ii. *Candida dubliniensis*
iii. *Candida tropicalis*
iv. *Candida parapsilosis*
v. *Candida glabrata*
vi. *Candida krusei*
*vii. Candida guilliermondii*
viii. *Candida auris*
comprises, consists essentially of or consists of the nucleotide sequence of SEQ ID NO: 3.

Described herein is at least one probe for detecting a yeast/fungus infection in a sample comprising a probe that hybridizes specifically to the ILV3 gene of the following *Aspergillus* species
i. *Aspergillus fumigatus*
ii. *Aspergillus niger*
iii. *Aspergillus flavus*

According to some embodiments at least one probe hybridises to at least 2, and preferably all 3, of the following target sequences:
i. positions 3721790-3721814 of nucleotide sequence accession number NC_007195.1 (*Aspergillus fumigatus*)
ii. positions c540811-540788 of nucleotide sequence accession number NT_166533.1 (*Aspergillus niger*)
iii. positions c382405-382381 of nucleotide sequence accession number NW_002477240.1 (*Aspergillus flavus*)
or the at least one probe hybridises to at least 2, and preferably all 3, of the following target sequences:
i. positions 3721848-3721870 of nucleotide sequence accession number NC_007195.1 (*Aspergillus fumigatus*)
ii. positions c540753-540731 of nucleotide sequence accession number NT_166533.1 (*Aspergillus niger*)
iii. positions c382347-382325 of nucleotide sequence accession number NW_002477240.1 (*Aspergillus flavus*).

In specific embodiments the probe that hybridizes specifically to the ILV3 gene of the following *Aspergillus* species
i. *Aspergillus fumigatus*
ii. *Aspergillus niger*
iii. *Aspergillus flavus*
comprises, consists essentially of or consists of the nucleotide sequence of SEQ ID NO: 72 or 75.

Described herein is at least one probe for detecting a yeast/fungus infection in a sample comprising a probe that hybridizes specifically to the ILV3 gene of *Candida albicans.*

According to some embodiments, the probe hybridizing specifically to the ILV3 gene of *Candida albicans* hybridises to one of the following target sequences from nucleotide sequence accession number NC_032093.1:
i. positions c1170174-1170151
ii. positions 1171165-1171192

In specific embodiments the probe hybridizing specifically to the ILV3 gene of *Candida albicans* comprises, consists essentially of or consists of the nucleotide sequence of SEQ ID NO: 116 or 117.

Described herein is at least one probe for detecting a yeast/fungus infection in a sample comprising a probe that hybridizes specifically to the ILV3 gene of *Candida dubliniensis.*

According to some embodiments, the probe hybridizing specifically to the ILV3 gene of *Candida dubliniensis* hybridises to one of the following target sequences from nucleotide sequence accession number NC_012864.1:
i. positions 1219148-1219171
ii. positions c1218439-1218416
iii. positions c1218505-1218480

In specific embodiments the probe hybridizing specifically to the ILV3 gene of *Candida dubliniensis* comprises, consists essentially of or consists of the nucleotide sequence of SEQ ID NO: 118, 119 or 120.

Described herein is at least one probe for detecting a yeast/fungus infection in a sample comprising a probe that hybridizes specifically to the ILV3 gene of *Candida tropicalis.*

According to some embodiments, the probe hybridizing specifically to the ILV3 gene of *Candida tropicalis* hybridises to the following target sequence from nucleotide sequence accession number NW_003020040.1:
i. positions c406111-406082

In specific embodiments the probe hybridizing specifically to the ILV3 gene of *Candida tropicalis* comprises, consists essentially of or consists of the nucleotide sequence of SEQ ID NO: 121.

Described herein is at least one probe for detecting a yeast/fungus infection in a sample comprising a probe that hybridizes specifically to the ILV3 gene of *Candida parapsilosis.*

According to some embodiments, the probe hybridizing specifically to the ILV3 gene of *Candida parapsilosis* hybridises to one of the following target sequences from nucleotide sequence accession number HE605203.1:
i. positions c24907-24883
ii. positions c24862-24839

In specific embodiments the probe hybridizing specifically to the ILV3 gene of *Candida parapsilosis* comprises, consists essentially of or consists of the nucleotide sequence of SEQ ID NO: 122 or 123.

Described herein is at least one probe for detecting a yeast/fungus infection in a sample comprising a probe that hybridizes specifically to the ILV3 gene of *Candida glabrata.*

According to some embodiments, the probe hybridizing specifically to the ILV3 gene of *Candida glabrata* hybridises to one of the following target sequences from nucleotide sequence accession number NC_005968.1:
i. positions c394151-394128
ii. positions c393478-393453
iii. positions c395030-395007
iv. positions c394836-394807

In specific embodiments the probe hybridizing specifically to the ILV3 gene of *Candida glabrata* comprises, consists essentially of or consists of the nucleotide sequence of SEQ ID NO: 124, 125, 126 or 127.

Described herein is at least one probe for detecting a yeast/fungus infection in a sample comprising a probe that hybridizes specifically to the ILV3 gene of *Candida krusei.*

According to some embodiments, the probe hybridizing specifically to the ILV3 gene of *Candida krusei* hybridises to one of the following target sequences from nucleotide sequence accession number JQFK01000016.1:
i. positions 61770-61793
ii. positions c60990-60967
iii. positions 60462-60485
iv. positions 61815-61838
v. positions c61970-61945

In specific embodiments the probe hybridizing specifically to the ILV3 gene of *Candida krusei* comprises, consists essentially of or consists of the nucleotide sequence of SEQ ID NO: 128, 129, 130, 131 or 132.

Described herein is at least one probe for detecting a yeast/fungus infection in a sample comprising a probe that hybridizes specifically to the ILV3 gene of *Candida guilliermondii.* According to some embodiments, the probe hybridizing specifically to the ILV3 gene of *Candida guilliermondii* hybridises to one of the following target sequences from nucleotide sequence accession number NW_001809800.1:
i. positions c909577-909552
ii. positions c911088-911065
iii. positions 910878-910904

In specific embodiments the probe hybridizing specifically to the ILV3 gene of *Candida guilliermondii* comprises, consists essentially of or consists of the nucleotide sequence of SEQ ID NO: 133, 134 or 135.

Described herein is at least one probe for detecting a yeast/fungus infection in a sample comprising a probe that hybridizes specifically to the ILV3 gene of *Candida auris.*

According to some embodiments, the probe hybridizing specifically to the ILV3 gene of *Candida auris* hybridises to one of the following target sequences from nucleotide sequence accession number NW_017263971.1:
i. positions c32744-32721
ii. positions c32421-32398
iii. positions c32629-32605
iv. positions 33233-33257
v. positions 32542-32563
vi. positions 32350-32373
vii. positions c33627-33606
viii. positions c32218-32196
ix. positions c32890-32864
x. positions c33259-33236
xi. positions c32061-32038

In specific embodiments the probe hybridizing specifically to the ILV3 gene of *Candida auris* comprises, consists essentially of or consists of a nucleotide sequence selected from SEQ ID NO: 136-146.

Described herein is at least one probe for detecting a yeast/fungus infection in a sample comprising a probe that hybridizes specifically to the ILV3 gene of *Aspergillus fumigatus.* According to some embodiments the probe hybridizing specifically to the ILV3 gene of *Aspergillus fumigatus* hybridises to one of the following target sequences from nucleotide sequence accession number NC_007195.1:
i. positions c3721651-3721628
ii. positions 3721658-3721681
iii. positions 3721848-3721870

In specific embodiments the probe that hybridizes specifically to the ILV3 gene of *Aspergillus fumigatus* comprises, consists essentially of or consists of the nucleotide sequence of SEQ ID NO: 78, 81 or 85.

Described herein is at least one probe for detecting a yeast/fungus infection in a sample comprising a probe that hybridizes specifically to the ILV3 gene of *Aspergillus niger*

According to some embodiments the probe hybridizing specifically to the ILV3 gene of *Aspergillus niger* hybridises to one of the following target sequences from nucleotide sequence accession number NT_166533.1:
i. positions c540161-540138.

In specific embodiments the probe that hybridizes specifically to the ILV3 gene of *Aspergillus niger* comprises, consists essentially of or consists of the nucleotide sequence of SEQ ID NO: 88.

Described herein is at least one probe for detecting a yeast/fungus infection in a sample comprising a probe that hybridizes specifically to the ILV3 gene of *Aspergillus flavus.*

According to some embodiments the probe hybridizing specifically to the ILV3 gene of *Aspergillus flavus* hybridises to one of the following target sequences from nucleotide sequence accession number NW_002477240.1:
i. positions c382572-382549

In specific embodiments the probe that hybridizes specifically to the ILV3 gene of *Aspergillus flavus* comprises, consists essentially of or consists of the nucleotide sequence of SEQ ID NO: 91.

Described herein is at least one probe for detecting a yeast/fungus infection in a sample comprising a probe that hybridizes specifically to the ILV3 gene of *Cryptococcus neoformans.*

According to some embodiments the probe hybridizing specifically to the ILV3 gene of *Cryptococcus neoformans* hybridises to one of the following target sequences from nucleotide sequence accession number NC_006693.1:
i. Positions 702724-702747
ii. Positions 702526-702549
iii. Positions 702805-702829
iv. Positions 702423-702446
v. Positions 701435-701458

In specific embodiments the probe that hybridizes specifically to the ILV3 gene of *Cryptococcus neoformans* comprises, consists essentially of or consists of the nucleotide sequence of SEQ ID NO: 95, 98, 101, 104 or 107.

The probes, as explained herein, are preferably used in combination for example in multiplex reactions. The invention provides sets of probes comprising at least two probes of the invention and which are intended to be used together (e.g. in a multiplex reaction and/or mastermix). Thus, in some embodiments, each probe is differentially labelled compared to the other probes (that are used in the amplification). As already discussed, many nucleic acid amplification protocols involve use of a probe. Examples include hydrolytic probes (e.g. TAQMAN probes) and hairpin probes (e.g. MOLECULAR BEACONS). Probes may also be attached to primers in some embodiments (e.g. SCORPION probes). Such probes may be differentially labelled as would be readily understood by one skilled in the art. This may involve inclusion of different fluorophores and/or different quenchers.

The primers and probes of the invention (which may be referred to as "detection components") are advantageously combined together to facilitate nucleic acid amplification based fungal detection, and characterisation in some embodiments. Preferred combinations of primer pairs and probes are set forth in Table B. Thus, the invention also provides a kit for detecting a yeast/fungus infection in a sample comprising at least one primer pair of the invention and/or at least one probe of the invention.

Kits containing pan-*Candida* and pan-*Aspergillus* detection components may be combined. They may be further combined with detection components to detect *Cryptococcus neoformans.* Thus, the kit may comprise combinations of primer pairs permitting detection of *Candida, Aspergillus* and *Cryptococcus neoformans.* The primer pairs may be provided in the form of a mastermix combination (i.e. a single master mix containing the primer pairs at suitable concentrations). Such kits may further comprise the relevant probes permitting detection of *Candida, Aspergillus* and *Cryptococcus neoformans.* The probes may also be included in the mastermix combination (again at a suitable concentration). One specific kit useful according to the invention comprises primers that comprise, consist essentially of or consist of the nucleotide sequences of SEQ ID NOs: 1 and 2 (for pan-*Candida* detection), SEQ ID NOs: 70 and 71 (for pan-*Aspergillus* detection) and SEQ ID NOs: 93 and 94 (for *Cryptococcus neoformans* detection). Such a kit may further comprise the probes of SEQ ID NOs: 3, 72 and 95 respectively.

Other kits of the invention, which may be combined with the kits described above, are useful for identifying the species responsible for a *Candida* infection in a sample. They contain appropriate *Candida* species specific primers of the invention. The primer pairs may be provided in the form of a mastermix combination (i.e. a single master mix containing the primer pairs at suitable concentrations). One specific kit useful according to the invention comprises primers that comprise, consist essentially of or consist of the nucleotide sequences of SEQ ID Nos 48 and 49, 18 and 19, 24 and 25, 40 and 41, 6 and 7, 8 and 9, 16 and 17 and 38 and 39 respectively.

Other kits of the invention, which may be combined with the kits described above, are useful for identifying the species responsible for an *Aspergillus* infection in a sample. They contain appropriate *Aspergillus* species specific primers of the invention. The primer pairs may be provided in the form of a mastermix combination (i.e. a single master mix containing the primer pairs at suitable concentrations). One specific kit useful according to the invention comprises primers that comprise, consist essentially of or consist of the nucleotide sequences of SEQ ID NOs 80 and 82, 86 and 87, 90 and 92.

The kits of the invention may contain various additional components. For example, they may contain reagents needed for amplification. They may contain one or more of a polymerase, dNTPs, MgCl₂, buffer etc. In some embodiments the kits may include DNA extraction reagents. More specifically, the kits may include reagents for extracting DNA from a blood sample. The kits may incorporate a suitable carrier in which the amplification reactions take place. Advantageously, such a carrier may comprise a multi-well plate, such as a 48 or 96 well plate for example. Such a carrier allows the detection methods to be carried out in relatively small volumes - thus facilitating scale up and minimising the sample volume required.

The kits will typically incorporate suitable instructions. These instructions permit the methods of the invention to be carried out reliably using the kits of the invention.

While particular primers and probes have been extensively described and are usefully applied in the methods of the invention, other primers and probes may be designed and applied to target ILV3. Accordingly, the invention provides a general method of detecting a fungal/yeast infection in a sample, comprising:
a. performing a nucleic acid amplification reaction to amplify the ILV3 gene of fungi/yeast
b. detecting the amplification product to determine whether the sample contains a fungal/yeast infection,
wherein the sample comprises a clinical sample obtained from a human subject.

According to all aspects of the invention ILV3 may be used to identify any fungus/yeast of interest. However, by "amplify the ILV3 gene" it is not intended that the entire ILV3 gene must be amplified. As the skilled person would be readily aware, only a portion of the ILV3 gene need be amplified to indicate the presence of the ILV3 gene. The minimum size of amplification product is typically governed by the primer length (and probe if included). Typical amplification products may be between 50 and 500 nucleotides in length, such as between 50 and 250 nucleotides. "Infection" simply refers to the presence of the fungus/yeast in a sample which ordinarily would not contain such fungus or yeast. Thus, the methods of the invention are also sensitive as well as specific to enable even low levels of fungal cells to be determined in the sample. A "sample" in the context of the present invention is a sample comprising a clinical sample obtained from a human subject. The sample may not, a *priori,* be known to contain a fungus. The sample is obtained from a human subject. The sample may, therefore, contain human genetic material (in particular human DNA). Thus the sample comprises a clinical sample, such as a blood sample. By blood sample is meant any sample comprising blood or a derivative thereof. Thus, serum and plasma are included together with blood broth (i.e. blood added to a culture medium). The methods of the invention are particularly applicable to the rapid detection and identification of the source of a fungal infection. Thus, the sample may comprise a blood culture sample from a patient suspected of suffering from, or being screened for, a bloodstream infection. The sample may be any suitable volume such as 1 to 10ml, preferably a 1ml blood culture sample.

The sample may comprise, consist essentially of or consist of tissue or cells and may comprise, consist essentially of or consist of a sputum or a blood sample or a platelet sample for example.

While the invention is applicable to potentially any fungus or yeast, there are particular fungi that are of importance to clinical diagnoses. Thus, the invention has been developed to target fungal genera and species that cause blood borne infections at relatively high frequency. Thus, the invention may focus on detection and optionally discrimination of *Candida* species. The invention may permit detection of all of the following species:
i. *Candida albicans*
ii. *Candida dubliniensis*
iii. *Candida tropicalis*
iv. *Candida parapsilosis*
v. *Candida glabrata*
vi. *Candida krusei*
vii. *Candida guilliermondii*
viii. *Candida auris*

This may be via pan-Candida targeting or by species-specific targeting of the ILV3 gene as explained in further detail herein. The invention may additionally, or alternatively, focus on detection and optionally discrimination of *Aspergillus* species. The invention may permit detection of at least 1, 2 or all 3 of the following species:
i. *Aspergillus fumigatus*
ii. *Aspergillus niger*
iii. *Aspergillus flavus*

This may be via pan-*Aspergillus* targeting or by species-specific targeting of the ILV3 gene as explained in further detail herein. The invention may additionally, or alternatively, focus on detection of *Cryptococcus neoformans.*

The invention thus provides a method of detecting a fungal/yeast infection in a sample, comprising:
a. performing a nucleic acid amplification reaction comprising the following components:
   i. a forward and reverse primer hybridizing specifically to the ILV3 gene of *Candida* species, optionally together with a probe that hybridizes between the primer binding sites specifically to the ILV3 gene of *Candida* species; and
   ii. a forward and reverse primer hybridizing specifically to the ILV3 gene of *Aspergillus* species, optionally together with a probe that hybridizes between the primer binding sites specifically to the ILV3 gene of *Aspergillus* species; and/or
   iii. a forward and reverse primer hybridizing specifically to the ILV3 gene of *Cryptococcus neoformans,* optionally together with a probe that hybridizes between the primer binding sites specifically to the ILV3 gene of *Cryptococcus neoformans*
b. detecting the amplification products to determine whether the sample contains a fungal/yeast infection,
wherein the sample comprises a clinical sample obtained from a human subject.

The methods of the invention may involve a nucleic acid amplification reaction that is capable of amplifying, in specific fashion, the ILV3 gene of at least 1, 2, 3, 4, 5, 6, 7, 8, 9, 10, 11 or all 12 of the following species:
i. *Candida albicans*
ii. *Candida dubliniensis*
iii. *Candida tropicalis*
iv. *Candida parapsilosis*
v. *Candida glabrata*
vi. *Candida krusei*
vii. *Candida guilliermondii*
viii. *Candida auris*
ix. *Aspergillus fumigatus*
x. *Aspergillus niger*
xi. *Aspergillus flavus*
xii. *Cryptococcus neoformans.*

The amplification used in the methods of the invention may involve use of a forward and reverse primer hybridizing specifically to the ILV3 gene of *Candida* species. Additionally or alternatively, the methods may involve use of a probe that hybridizes specifically to the ILV3 gene of *Candida* species. In some embodiments, a common forward and reverse primer and/or common probe hybridises to the ILV3 gene of at least 2, 3, 4, 5, 6, 7 and preferably all, of the following *Candida* species:
i. *Candida albicans*
ii. *Candida dubliniensis*
iii. *Candida tropicalis*
iv. *Candida parapsilosis*
v. *Candida glabrata*
vi. *Candida krusei*
vii. *Candida guilliermondii*
viii. *Candida auris*

Thus, in some embodiments, pan-*Candida* amplification is utilised. Suitable primers and probes of the invention for pan-*Candida* amplification are described herein, including specific targeting regions within ILV3, and all such primers and probes may be utilised. In one embodiment, the methods of the invention comprise use of a forward primer comprising the sequence of SEQ ID NO: 1, a reverse primer comprising the sequence of SEQ ID NO: 2 and/or a probe comprising the sequence of SEQ ID NO: 3.

In other embodiments, including methods for discriminating the source of the infection, *Candida* species specific amplification is adopted. In such embodiments, a separate forward and reverse primer and/or probe hybridises to the ILV3 gene of each of at least 2, 3, 4, 5, 6, 7 and preferably all, of the following *Candida* species:
i. *Candida albicans*
ii. *Candida dubliniensis*
iii. *Candida tropicalis*
iv. *Candida parapsilosis*
v. *Candida glabrata*
vi. *Candida krusei*
vii. *Candida guilliermondii*
viii. *Candida auris*

Thus, there is a separate primer pair and/or probe for each *Candida* species to be detected. Suitable primers and probes of the invention for species specific *Candida* amplification are described herein, including specific targeting regions within ILV3, and all such primers and probes may be utilised.

The amplification used in the methods of the invention may additionally or alternatively involve use of a forward and reverse primer hybridizing specifically to the ILV3 gene of *Aspergillus* species. Additionally or alternatively, the methods may involve use of a probe that hybridizes specifically to the ILV3 gene of *Aspergillus* species. In some embodiments, a common forward and reverse primer and/or common probe hybridises to the ILV3 gene of at least 2, and preferably all 3, of the following *Aspergillus* species:
i. *Aspergillus fumigatus*
ii. *Aspergillus niger*
iii. *Aspergillus flavus*

Thus, in some embodiments, pan- *Aspergillus* amplification is utilised. Suitable primers and probes of the invention for pan- *Aspergillus* amplification are described herein, including specific targeting regions within ILV3, and all such primers and probes may be utilised. In one embodiment, the methods of the invention comprise use of a forward primer comprising the sequence of SEQ ID NO: 70 or 73, a reverse primer comprising the sequence of SEQ ID NO: 71 or 74 and/or a probe comprising the sequence of SEQ ID NO: 72 or 75.

In other embodiments, including methods for discriminating the source of the infection, *Aspergillus* species specific amplification is adopted. In such embodiments, a separate forward and reverse primer and/or probe hybridises to the ILV3 gene of each of at least 2, and preferably all 3, of the following *Aspergillus* species:
i. *Aspergillus fumigatus*
ii. *Aspergillus niger*
iii. *Aspergillus flavus.*

Thus, there is a separate primer pair and/or probe for each *Aspergillus* species to be detected. Suitable primers and probes of the invention for species specific *Aspergillus* amplification are described herein, including specific targeting regions within ILV3, and all such primers and probes may be utilised.

The amplification used in the methods of the invention may additionally or alternatively involve use of a forward and reverse primer hybridizing specifically to the ILV3 gene of *Cryptococcus neoformans.* Additionally or alternatively, the methods may involve use of a probe that hybridizes specifically to the ILV3 gene of *Cryptococcus neoformans.* Suitable primers and probes of the invention for *Cryptococcus neoformans* amplification are described herein, including specific targeting regions within ILV3, and all such primers and probes may be utilised.

According to the methods of the invention, amplification products can simply be detected to indicate a fungal species is present in the sample. Amplification products can be detected via any known means as would be readily appreciated by one skilled in the art. In some embodiments, however, discrimination of amplification products is used in order to identify the genus and/or species of the fungus present in the sample. Thus, methods of the invention may involve detecting and identifying a fungal/yeast infection in a sample. They may comprise performing the nucleic acid amplification and detecting and distinguishing the amplification products to identify the fungal/yeast infection.

The invention therefore provides a method of detecting and identifying a fungal/yeast infection in a sample, comprising:
a. performing a nucleic acid amplification reaction comprising the following components:
   i. a forward and reverse primer hybridizing specifically to the ILV3 gene of *Candida* species, optionally together with a probe that hybridizes between the primer binding sites specifically to the ILV3 gene of *Candida* species; and
   ii. a forward and reverse primer hybridizing specifically to the ILV3 gene of *Aspergillus* species, optionally together with a probe that hybridizes between the primer binding sites specifically to the ILV3 gene of *Aspergillus* species; and/or
   iii. a forward and reverse primer hybridizing specifically to the ILV3 gene of *Cryptococcus neoformans,* optionally together with a probe that hybridizes between the primer binding sites specifically to the ILV3 gene of *Cryptococcus neoformans*
b. detecting and distinguishing the amplification products to identify the fungal/yeast infection,
wherein the sample comprises a clinical sample obtained from a human subject.

The methods of the invention that permit the identity of the fungus in the sample to be identified are particularly useful to direct treatment in a manner that is specific for the infection at hand. They are thus useful separately from general fungal detection methods. Thus, the invention also relates to methods of identifying the species responsible for a *Candida* infection in a sample, comprising:
a. performing nucleic acid amplification reactions using at least three, 4, 5, 6, 7 or all of the following sets of components:
   i. a forward and reverse primer hybridizing specifically to the ILV3 gene of *Candida albicans*
   ii. a forward and reverse primer hybridizing specifically to the ILV3 gene of *Candida dubliniensis*
   iii. a forward and reverse primer hybridizing specifically to the ILV3 gene of *Candida tropicalis*
   iv. a forward and reverse primer hybridizing specifically to the ILV3 gene of *Candida parapsilosis*
   v. a forward and reverse primer hybridizing specifically to the ILV3 gene of *Candida glabrata*
   vi. a forward and reverse primer hybridizing specifically to the ILV3 gene of *Candida krusei*
   vii. a forward and reverse primer hybridizing specifically to the ILV3 gene of *Candida guilliermondii*
   viii. a forward and reverse primer hybridizing specifically to the ILV3 gene of *Candida auris*
b. detecting and distinguishing the amplification products to identify the species responsible for the *Candida* infection,
wherein the sample comprises a clinical sample obtained from a human subject.

These methods may employ any of the suitable primers (and probes) of the invention that are described herein in detail.

Similarly, the invention also provides a method of identifying the species responsible for an *Aspergillus* infection in a sample, comprising:
a. performing nucleic acid amplification reactions using at least two or all three of the following sets of components:
   i. a forward and reverse primer hybridizing specifically to the ILV3 gene of *Aspergillus fumigatus*
   ii. a forward and reverse primer hybridizing specifically to the ILV3 gene of *Aspergillus niger*
   iii. a forward and reverse primer hybridizing specifically to the ILV3 gene of *Aspergillus flavus*
b. detecting and distinguishing the amplification products to identify the species responsible for the *Aspergillus* infection,
wherein the sample comprises a clinical sample obtained from a human subject.

These methods may employ any of the suitable primers (and probes) of the invention that are described herein in detail. These methods may be employed in parallel with *Candida* identification methods in some embodiments.

The invention provides methods that can be considered a general screen to determine whether a fungal infection is present. The invention also provides more specific methods that permit the species responsible for the infection to be identified. Such methods may advantageously be combined. Accordingly, the invention further provides a method of detecting and identifying a yeast/fungal infection in a sample comprising:
a. Performing a method of the invention in order to determine whether *Candida, Aspergillus* and/or *Cryptococcus neoformans* is present in the sample
b. In the event that a species of *Candida* or *Aspergillus* is present in the sample performing a method of the invention in order to determine which species is present
to thereby detect and identify the yeast/fungal infection in the sample,
wherein the sample comprises a clinical sample obtained from a human subject.

In step a, the detection of *Cryptococcus neoformans* may remove the requirement to perform step b. Alternatively, the outcome of step a may simply be that there is a fungus present in the sample. In such methods, step b may additionally comprise performing a method of the invention to determine whether *Cryptococcus neoformans* specifically is present in the sample.

As discussed herein, the methods of the invention generally involve nucleic acid amplification of the ILV3 gene. Any form of nucleic acid amplification can be used, although polymerase chain reaction (PCR) is preferred. Such methods may employ any suitable form of detection technology. Real-time monitoring of amplification may be used in some embodiments. In other embodiments, an end-point detection method may be employed. In some embodiments, the nucleic acid amplification is performed as a multiplex nucleic acid amplification reaction. In other embodiments, each nucleic acid amplification targeting ILV3 from a different genus or species is included in a separate reaction area. A reaction area is a defined location at which amplification takes place. It may be a well in a multi-well plate or a test tube for example. Sequencing, in particular next generation sequencing (NGS), may be utilised for detection and optionally also discrimination. Examples of NGS platforms include Illumina sequencing (such as Hi-Seq and Mi-Seq), SMRT sequencing (Pacific Biosciences), Nanopore sequencing, SoLID sequencing, pyrosequencing (e.g. Roche 454), single molecule sequencing (SeqLL/Helicos) and Ion-Torrent (Thermo Fisher) which are well-known to the skilled person and commercially available. As described herein, the ILV3 gene from different fungi has a different nucleotide sequence which can be probed using sequencing to identify the source of a fungal infection. Sequencing may provide rapid and quantitative results.

For simple detection, the mere presence of an amplification product indicates that there is a fungus present in the sample. As discussed herein, this is typically a fungus selected from *Candida, Aspergillus* and *Cryptococcus neoformans.* However, where more detailed information on the nature of the fungus is required the amplification products may be distinguished. In some embodiments, distinguishing involves a melt curve analysis. Various primer pairs described herein have been designed to have non-overlapping melt curves. Thus, when included in a multiplex amplification, the melt curve generated permits the species of *Candida* or *Aspergillus* in the sample to be identified. This may be a separate multiplex for *Candida* to the multiplex used for *Aspergillus* discrimination. It is shown herein that the methods of the invention based on a melt curve analysis permit discrimination of 8 different species of *Candida* and 3 different species of *Aspergillus* respectively. Melt curve analysis according to the invention may or may not rely upon use of sequence specific probes. In preferred embodiments, the methods do not require use of ILV3 specific probes. Instead a sequence independent reagent such as an intercalating agent, one example of which is SYBR GREEN, may be used to monitor amplification.

In other embodiments, amplification products may be distinguished by using differentially labelled primers and/or probes. In some embodiments, at least one primer and/or probe is differentially labelled according to genus to permit identification of the genus of fungus/yeast in the sample. In some embodiments, at least one primer and/or probe is differentially labelled according to species of *Candida* and/or *Aspergillus* to permit identification of the species of *Candida* and/or *Aspergillus* in the sample.

In still further embodiments, amplification products may be distinguished by determining the size of the amplification products. Primer pairs can be designed to amplify differently sized amplification products within the ILV3 gene of different genera and species if required.

In other embodiments, amplification products may be distinguished according to sequence.

The invention can advantageously be implemented in order to also detect bacteria in a sample. More specifically, the methods may further permit the determination of whether a bacteria or a fungus is present in the sample. In some embodiments, the methods may permit distinguishing whether the bacteria is Gram positive or Gram negative. Suitable reagents for such methods of detecting bacteria are disclosed in Klaschik et al (J. Clin. Microbiol. 2002, 40(11):4304) and Wu et al (JOURNAL OF CLINICAL MICROBIOLOGY, Aug. 2008, p. 2613-2619).

Such methods may rely upon use of a probe to distinguish Gram-negative from Gram-positive bacteria. In some embodiments, the primers amplify specific parts of the 16S region of bacterial DNA. The primers PLK1 (5-TACGGGAGGCAGCAGT-3 - SEQ ID NO: 108) and PLK2 (5-TATTACCGC GGCTGCT-3 - SEQ ID NO: 109) are highly conserved in different groups of eubacteria. A 187-bp fragment is synthesized by these primers. PLK2 may be labelled with fluorescein internally. The fluorescence dye-labelled hybridization probes ISN2 (5-CCGCAGAATAAG CACCGGCTAACTCCGT-3 - SEQ ID NO: 110) and ISP2 (5-CCT AAC CAG AAA GCC ACG GCT AAC TAC GTG-3 - SEQ ID NO: 111) emit light at different wavelengths (640 and 705 nm) and can be used for detection and Gram stain differentiation of bacterial DNA by a fluorescence signal. Other suitable primers may comprise the nucleotide sequence CAACGCGAAGAACCTTACC (SEQ ID NO: 112) and ACGTCATCCCCACCTTCC (SEQ ID NO: 113). A suitable Gram-positive probe comprises the nucleotide sequence 5'-FAM-ACGACAACCATGCACCACCTG-TAMRA-3' (SEQ ID NO: 114). A suitable Gram-negative probe comprises the nucleotide sequence 5'-HEX-ACGACAGCCATGCAGCACCT-TAMRA'3 (SEQ ID NO: 115). Although these probes are differently labelled to permit differential detection, it will be appreciated by the skilled person that alternative approaches as described herein may be adopted to facilitate detection.

Thus, the invention further provides a method of detecting and identifying a microbial infection in a sample, comprising:
a. performing a nucleic acid amplification reaction comprising the following components:
   i. a forward and reverse primer hybridizing specifically to the 16S rRNA gene of Gram positive bacteria; optionally together with a probe that hybridizes between the primer binding sites specifically to the 16S rRNA gene of gram positive bacteria
   ii. a forward and reverse primer hybridizing specifically to the 16S rRNA gene of Gram negative bacteria; optionally together with a probe that hybridizes between the primer binding sites specifically to the 16S rRNA gene of Gram negative bacteria
   iii. a forward and reverse primer hybridizing specifically to the ILV3 gene of at least one fungal/yeast species; optionally together with a probe that hybridizes between the primer binding sites specifically to the ILV3 gene of at least one fungal/yeast species
b. detecting and distinguishing the amplification products to determine whether the sample contains a Gram negative bacterial infection, a Gram positive bacterial infection and/or a fungal/yeast infection
wherein the sample comprises a clinical sample obtained from a human subject.

Preferably, the amplification is performed as a multiplex although this is not essential as explained herein. ILV3 amplification may be performed according to any method of the invention or using any of the relevant primers and/or probes of the invention. Corresponding kits are also provided. Thus, the invention provides a kit for discriminating a microbial infection in a sample, comprising components for performing a multiplex nucleic acid amplification reaction comprising:
a. a forward and reverse primer hybridizing specifically to the 16S rRNA gene of Gram positive bacteria; optionally together with a probe that hybridizes between the primer binding sites specifically to the 16S rRNA gene of Gram positive bacteria
b. a forward and reverse primer hybridizing specifically to the 16S rRNA gene of Gram negative bacteria; optionally together with a probe that hybridizes between the primer binding sites specifically to the 16S rRNA gene of Gram negative bacteria and
c. a forward and reverse primer hybridizing specifically to the ILV3 gene of at least one fungal/yeast species; optionally together with a probe that hybridizes between the primer binding sites specifically to the ILV3 gene of at least one fungal/yeast species;
wherein components a, b and c each produce distinguishable amplification products thus enabling a determination of whether the sample contains a Gram negative bacterial infection, a Gram positive bacterial infection and/or a fungal/yeast infection.

Any suitable primer and probe according to the invention may be incorporated into such kits together with primers and probes for 16S rRNA amplification primers and probes. All embodiments of the invention discussed herein apply *mutatis mutandis* to these aspects of the invention. These methods may be followed by fungal species identification where needed. The kits may contain suitable components for this purpose as described herein. The invention effectively provides for patient selection for therapy and, critically, avoids unnecessary treatment with antifungal agents such as fungicides (or antibiotics if bacteria are also detected). Incorrect use of antifungal agents and antibiotics fuels resistance. Accordingly, the invention also relates to a method of selecting a subject for treatment with an antifungal agent such as a fungicide (or an antibiotic if bacteria are detected) comprising performing a method described herein and selecting the subject for treatment where an infection is detected, optionally also identified.

In a related aspect, the present invention provides a method of predicting responsiveness of a subject to treatment with an antifungal agent such as a fungicide (or an antibiotic if bacteria are detected) comprising performing a method described herein and predicting responsiveness of the subject to treatment where an infection is detected, optionally also identified.

In a further aspect which is not part of the invention, provided is a method of treating an infection comprising administering an antifungal agent such as a fungicide (or an antibiotic if bacteria are detected) to the subject suffering from the infection, wherein the subject has been selected for treatment by performing a method described herein.

In yet a further aspect, the present invention provides an antifungal agent such as a fungicide for use in a method of treating an infection, wherein the subject has been selected for treatment by performing the method described herein.

According to a further aspect of the invention there is provided an antifungal agent such as a fungicide for use in a method of treating an infection, wherein the subject displays, in a sample, a detectable ILV3 gene, and wherein the sample comprises a clinical sample obtained from a human subject.

The infection may be a fungal or yeast infection, in particular a *Candida, Aspergillus* or *Cryptococcus neoformans* infection as explained herein in greater detail. This may direct the specifics of the treatment provided. For example, *C. auris* has been shown to be resistant to three main classes of antifungal drugs, including azoles (e.g. fluconazole). Similarly, species such as *C. glabrata* and *C. krusei,* may have a decreased susceptibility to anti-fungal agents such as fluconazole relative to other *Candida* species (Trick *et al.,* 2002).

In certain embodiments the antifungal agent such as a fungicide is a broad spectrum agent. This is particularly useful if an infection is detected but where the species responsible for the infection has not yet been characterised. Once the infection has been detected, the nature of the infection may be characterised so as to allow more targeted therapy (e.g. the species of *Candida* causing the infection). Thus, combinations of broad spectrum antifungal agents such as a fungicide (or an antibiotic if bacteria are detected) and more focussed therapies may be employed as part of the methods described herein.

### DESCRIPTION OF THE FIGURES

Figure 1 shows amplification curves in which the identification of 8 different Candida species was achieved by using a single primer-probe set containing degenerate bases (pan-Candida set).
Figure 2 is an overlay of the melt curves from multiple Candida species using the best single primer-probe set for each species.
Figures 3A (set 1) and 3B (set 2) show amplification curves in which two pan-*Aspergillus* primer-probe sets yielded amplification for the three *Aspergillus* species tested.
Figures 4A and 4B show amplification curves from experiments to determine which primer-probe sets gave maximal performance, in terms of (a low) Ct detection value and (a high) level of fluorescence at the end of the amplification protocol.

### DETAILED DESCRIPTION (EXPERIMENTAL EXAMPLES)

The *ILV3* gene represents a novel gene for the detection of *Candida* and other fungal organisms (including *Aspergillus* spp. and *Cryptococcus neoformans*). The *ILV3* gene, which encodes for di-hydroxyacid dehydratase, an enzyme that catalyses the third step in the common pathway leading to biosynthesis of branched-chain amino acids, is a yeast/fungal-specific gene with 0% (zero) homology to any human DNA (Liu *et al.,* 2006). Current fungal detection methods focus on ribosomal DNA (either 18S, internal transcribed spacers (5.8S) or 28S rRNA), which have large regions of homology to equivalent genes found in humans (ref: Khot *et al.,* 2009; and Kan, 1992). This high level of homology makes the generation of specific primers and detection probes both challenging and time consuming as primer-probe sets need to be checked for their cross-reactivity to human DNA. Also, considering the products and methods of the invention will often use as the test sample lysates derived from human blood samples, any residual human DNA will be randomly sheared which may increase the likelihood of a human DNA sequence having complementarity, and thus cross-reactivity, to a ribosomal rRNA-based fungal primer-set. In contrast, with the use of an *ILV3*-based primer-probe set this screening process is not needed, and the risk of cross-reactivity to human DNA is eliminated. In parallel to targeting *ILV3,* it was also decided to employ hydrolysis ("TAQMAN") probes for sample detection. TAQMAN probes allow for excellent sensitivity, specificity and qPCR performance to be achieved. However, in other embodiments, alternative probe types may be used. For example MOLECULAR BEACONS or SCORPIONS may be used to target *ILV3* for the detection of yeast and fungi.

In addition to targeting *ILV3* for the detection of *Candida,* this gene can also be targeted for, but not limited to, the detection of other fungal pathogens including several species of *Aspergillus,* as well as *Cryptococcus neoformans.*

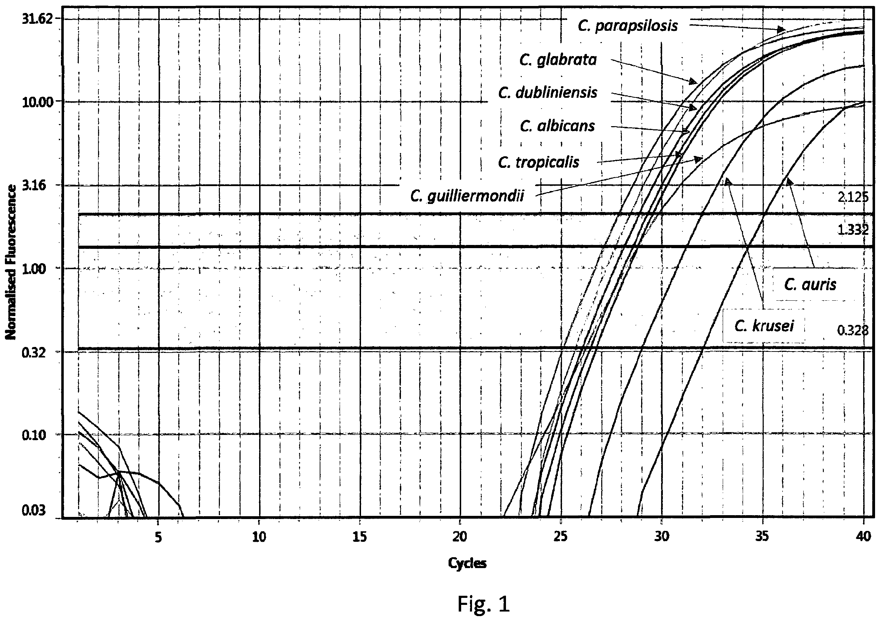

For the identification of *Candida,* bioinformatic analysis of the *ILV3* gene (through sequence alignment of this gene against multiple *Candida* species) identified two regions that could be used for the detection of *Candida* species. Experiments demonstrated that the identification of multiple (8) species could be achieved, by using a single primer-probe set, which contained degenerate bases (a 'pan-*Candida*' primer-probe set; Primer-probe Set2 -See Table 1 and Figure 1.

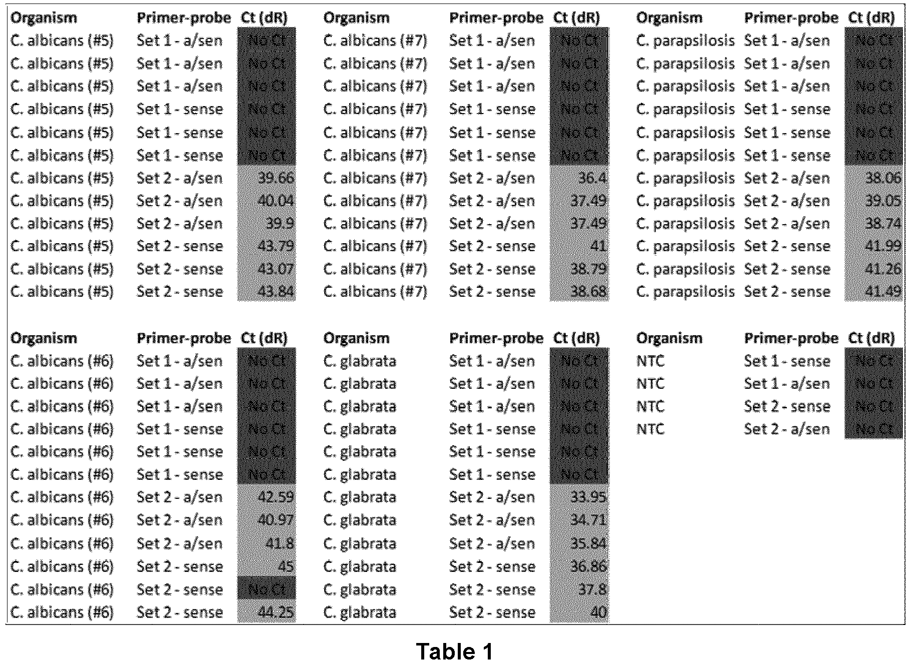

In order to further increase the performance of *Candida* detection (to enhance sensitivity and specificity) numerous variants of the original primer-probe set were designed and tested by substituting bases within the sequence of the primers and probe. The modifications to the reverse primer sequence and the probe sequence did not have any beneficial effect (data not shown). However, one of the re-designed forward primers enhanced the sensitivity of detection of a previously detectable *Candida* species (i.e. lower Ct (Cp) value relative to the original forward primer - an increase in the Delta Ct), as well as enhancing the performance specificity of detecting *Candida guilliermondii,* which previously had poor detection with the original primer-probe design - see Table 2. This new, updated set (SEQ ID NO: 1-3) has now been adopted (sequences shown in Table B).

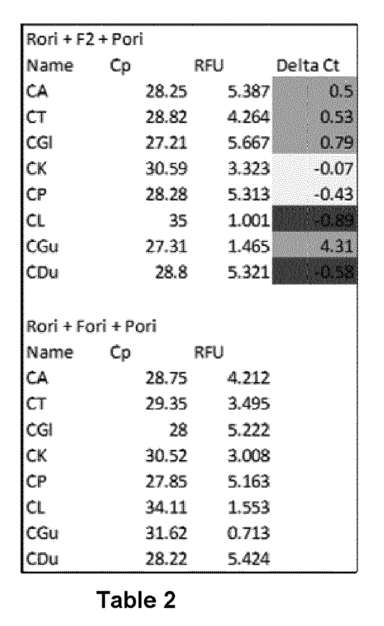

A recent Public Health England report lists the individual species of *Candida* associated with candidaemia ("Surveillance of candidaemia in England, Wales and Northern Ireland, 2014"). From this report, it was decided to target the following *Candida* species whose genomes had been sequenced and published: *C. albicans, C. glabrata, C. parapsilosis, C. tropicalis, C. krusei, C. dubliniensis,* and C. guilliermondii. In addition to these species, *Candida auris,* the eighth species, was also added to the above list of organisms to be targeted for species-specific identification. The reason for this is because this species has been identified by Public Health England ("*Candida auris* identified in England") as a significant emerging fungal pathogen with sporadic cases of *C. auris* having been identified throughout England and other countries Worldwide. Furthermore, *C. auris* has been shown to have a propensity for transmission between hospital patients, as well as showing resistance to three main classes of antifungal drugs, including azoles (e.g. fluconazole). Additional longitudinal epidemiological data has shown a recent shift in the incidence of bloodstream infections caused by non-*albicans* species of *Candida* (Wisplinghoff *et al.,* 2014). These species, for example *C. glabrata* and *C. krusei,* have a decreased susceptibility to anti-fungal agents such as fluconazole relative to other *Candida* species (Trick *et al.,* 2002). Therefore, there is a clinical need to be able to discriminate and differentiate between these eight most prevalent *Candida* species, especially *C auris.*

To achieve this level of differentiation, the *ILV3* gene was again interrogated bioinformatically. Multiple primer sets were designed for each species individually, with bioinformatic analysis of each primer set being performed to determine the specificity of these primer sets to the species of interest and ensuring no homology (cross-reactivity) was seen to other *Candida* species. The resulting primer sets were tested by melt curve analysis, using SYBR Green chemistry. Each primer set would generate an amplified region of DNA ('amplicon') of a precise melting temperature (Tm). Experiments using all the primer sets were conducted in order to determine the Tm profile of each primer set, for each *Candida* species - see Table 3.

**Table 3**

| **Candida species** | **SEQ IDs** | **Actual TM (°C)** | **St. dev (°C)** |
|---|---|---|---|
| *C. albicans* | 4,5 | 80.9 | 0.04 |
| | 6,7 | 78.51 | 0.01 |
| *C. dubliniensis* | 8,9 | 77.56 | 0 |
| | 10,11 | 79.6 | 0.04 |
| | 12,13 | 80.55 | 0.01 |
| *C. tropicalis* | 14,15 | 76.93 | 0 |
| | 16,17 | 76.33 | 0.01 |
| C. *parapsilosis* | 18,19 | 81.03 | 0.1 |
| | 20,21 | 77.38 | 0 |
| *C. glabrata* | 22, 23 | 79.27 | 0.12 |
| | 24, 25 | 79.79 | 0.04 |
| | 26,27 | 80.39 | 0.08 |
| | 28,29 | 76.6 | 0 |
| *C. krusei* | 30,31 | 78.76 | 0.06 |
| | 32,33 | 79.27 | 0.06 |
| | 34,35 | 80.35 | 0 |
| | 36,37 | 79.81 | 0 |
| | 38,39 | 75.41 | 0.01 |
| *C. guilliermondii* I | 40,41 | 78.96 | 0.01 |
| | 42,43 | 80.55 | 0 |
| | 44,45 | 80.52 | 0.01 |
| | 46,47 | 78.35 | 0 |
| *C. auris* | 48,49 | 82.13 | 0.01 |
| | 50,51 | 79.81 | 0.04 |
| | 52,53 | 79.91 | 0.01 |
| | 54,55 | 80.39 | 0.04 |
| | 56,57 | 83.09 | 0.02 |
| | 58,59 | 78.92 | 0.02 |
| | 60,61 | 82.75 | 0 |
| | 62,63 | 78.06 | 0.01 |
| | 64,65 | 78.87 | 0.01 |
| | 66,67 | 81.74 | 0 |
| | 68,69 | 81.86 | 0.05 |

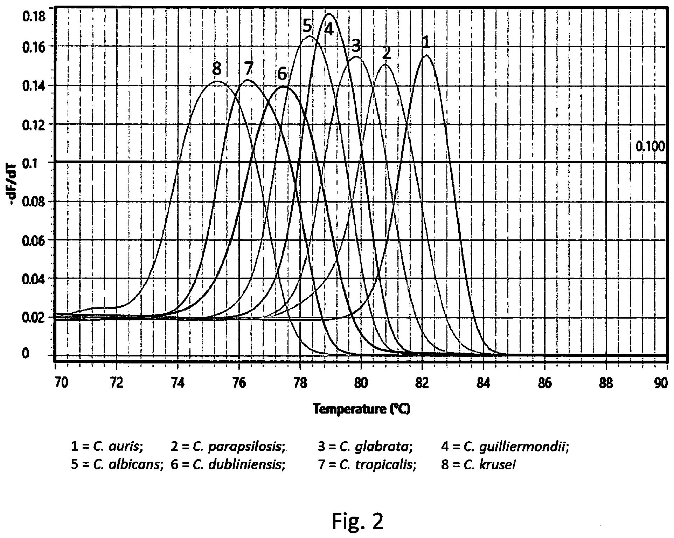

Once completed, the best single primer-probe set for each species was selected to optimise spread and separation between the Tm values of each species - see Table 4. When these primer sets were again tested experimentally, and the melt curve trace of each species was overlaid, there was very good resolution between the eight traces, showing that this approach is a valid way of discriminating between individual *Candida* species - see Figure 2. These primer sets provide delineation of more *Candida* species than the current test from T2 Biosystems.

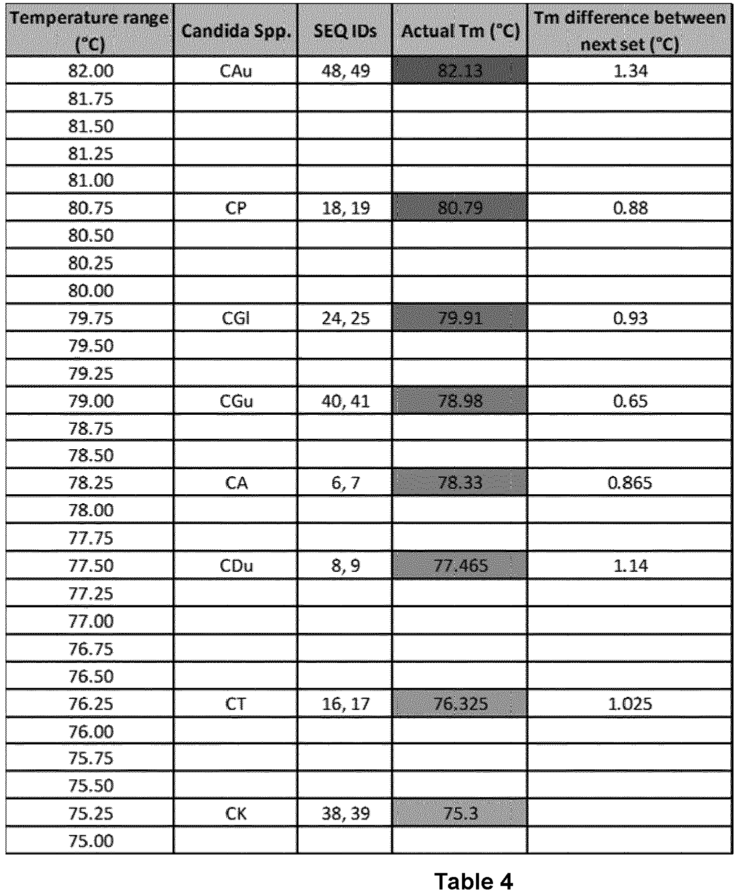

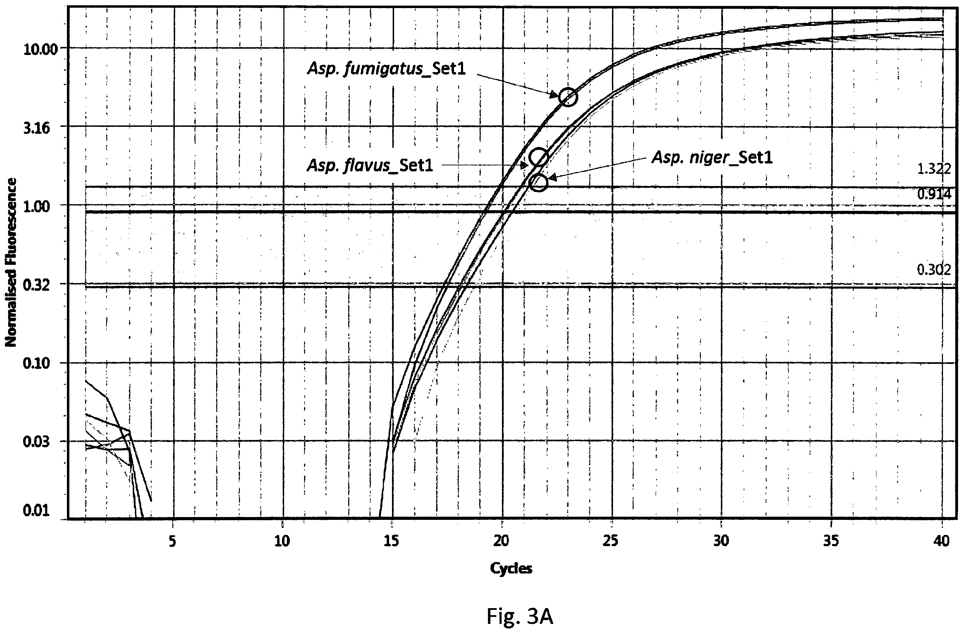

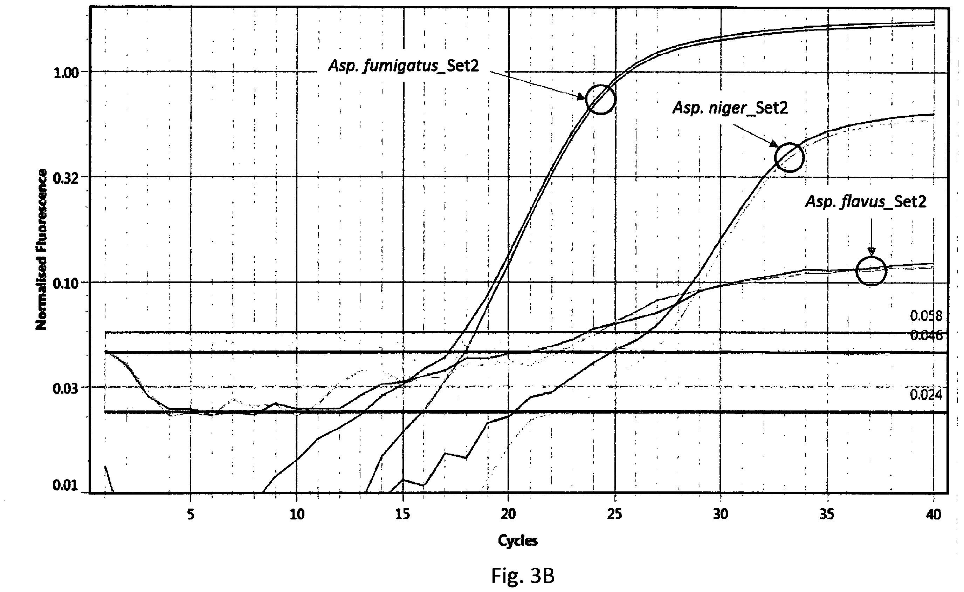

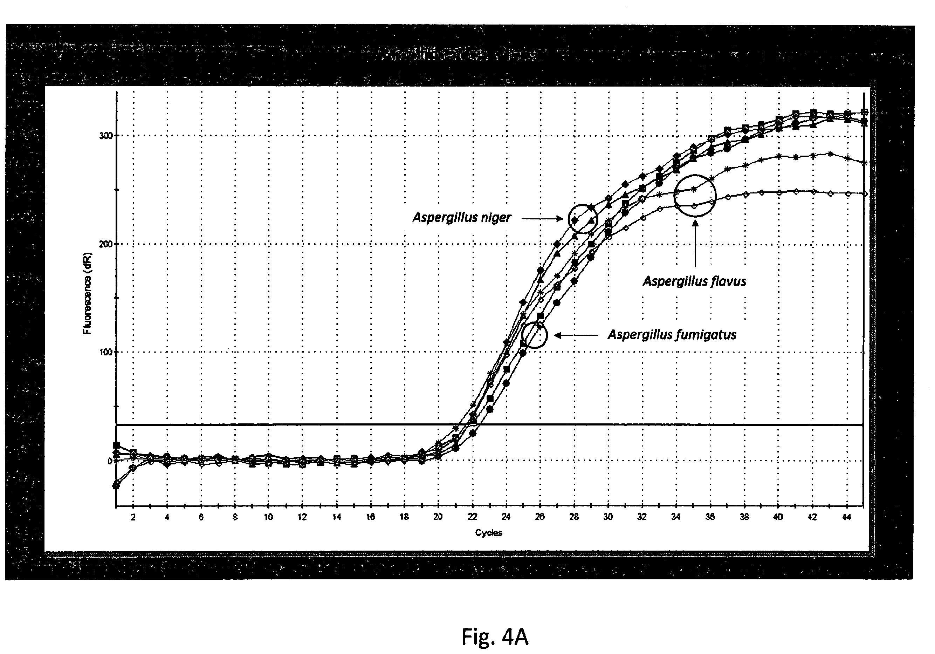

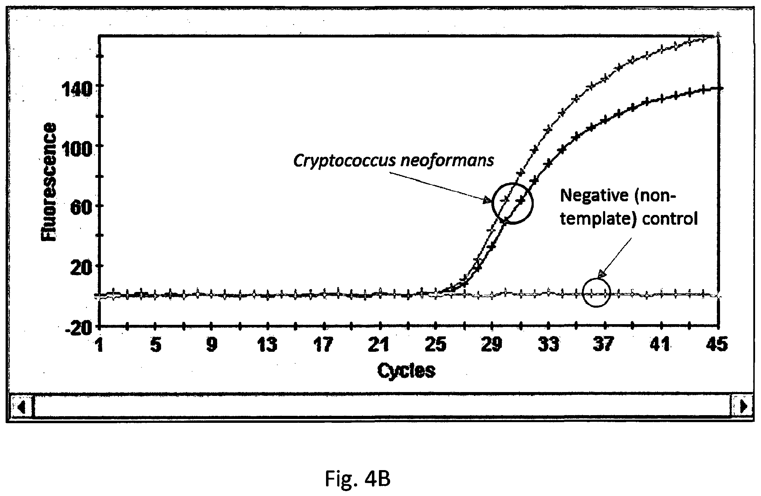

In addition to the detection of *Candida* species, *ILV3* can also be used as a target for the detection of other fungal organisms such as *Aspergillus* species (particularly focused on *Aspergillus fumigatus, Asp. niger,* and *Asp. flavus*) and *Cryptococcus neoformans.* In an identical approach, as outlined previously for *Candida,* bioinformatic analysis and sequence alignment, where applicable, was used to identify suitable regions within the *ILV3* gene for the design of a 'pan-*Aspergillus'* primer-probe set as well as several primer-probe sets for species-specific identification of the three *Aspergillus* species, as well as *C. neoformans.* Experiments demonstrated that two pan-*Aspergillus* primer-probe sets yielded amplification for the three *Aspergillus* species tested, but that the V1 design (SEQ ID Nos 70-72; Figure 3A) was preferred due to better fluorescence across these species - see Figures 3A and 3B. For the generation of species-specific primer-probe sets, various design iterations for each species were tested bioinformatically to demonstrate species specificity (especially important for the detection of different *Aspergillus* species) as well as ensuring no homology to other fungal organisms or human genes. Primer-probe sets that passed this validation approach were then tested *in vitro* to determine which set gave maximal performance, in terms of (a low) Ct detection value and (a high) level of fluorescence at the end of the amplification protocol - see Tables 5-6. Figure 4A shows amplification traces of the chosen species-specific primer-probe set for the three *Aspergillus* species (*Asp. fumigatus*: SEQ ID NO: 79-81; *Asp. niger*: SEQ ID NO: 86-88; and *Asp. flavus*: SEQ ID NO: 89-91. See Table B for sequences). Figure 4B shows the amplification trace of the chosen primer-probe set for the detection of *Cryptococcus neoformans* (SEQ ID NO: 93-95).

**Table 5**

| **SEQ IDs** | **Ct** | **Av. Ct** | **EndPt** | **Av. EndPt** |
|---|---|---|---|---|
| 76, 77, 78 | 22.05 | 22.24 | 123.107 | 152.723 |
| | 22.43 | | 182.338 | |
| 79, 80, 81 | 22.96 | 22.65 | 345.802 | 331.714 |
| | 22.34 | | 317.626 | |
| 83, 84, 85 | 22.28 | 22.66 | 266.964 | 228.686 |
| | 23.03 | | 190.408 | |

**Table 6**

| **SEQ IDs** | **Sample ID** | **Ct** | **EndPt** |
|---|---|---|---|
| 93, 94, 95 | CN-1 | 27.2 | 138 |
| | CN-1 | 26.9 | 174 |
| | NTC-1 | 0 | 0 |
| 96, 97, 98 | CN-2 | 29.2 | 142 |
| | CN-2 | 29.2 | 133 |
| | NTC-2 | 0 | 0 |
| 99, 100, 101 | CN-3 | 27.5 | 81 |
| | CN-3 | 27.1 | 92 |
| | NTC-3 | 0 | 0 |
| 102, 103, 104 | CN-4 | 27.6 | 69 |
| | CN-4 | 27.5 | 60 |
| | NTC - 4 | 0 | 1 |
| 105, 106, 107 | CN-5 | 26.9 | 72 |
| | CN-5 | 26.7 | 77 |
| | NTC -5 | 0 | -1 |

Alongside this, primer sets for melt curve analysis were tested to differentiate between the three *Aspergillus* species mentioned above. Again, the same bioinformatics approach used for the identification of *Candida* melt primers was used in order to find suitable *Aspergillus* melt primers. When these *Aspergillus* melt primers were tested *in vitro* a good resolution between the Tm value of each of the three *Aspergillus* species was achieved - see Tables 7-8.

**Table 7**

| **Aspergillus species** | **SEQ IDs** | **Actual Tm (°C)** | **St. dev (°)** |
|---|---|---|---|
| *Asp. fumigatus* | 80, 82 | 82.95 | 0.01 |
| *Asp. niger* | 86, 87 | 79.54 | 0.01 |
| *Asp. flavus* | 90, 92 | 81.82 | 0.01 |

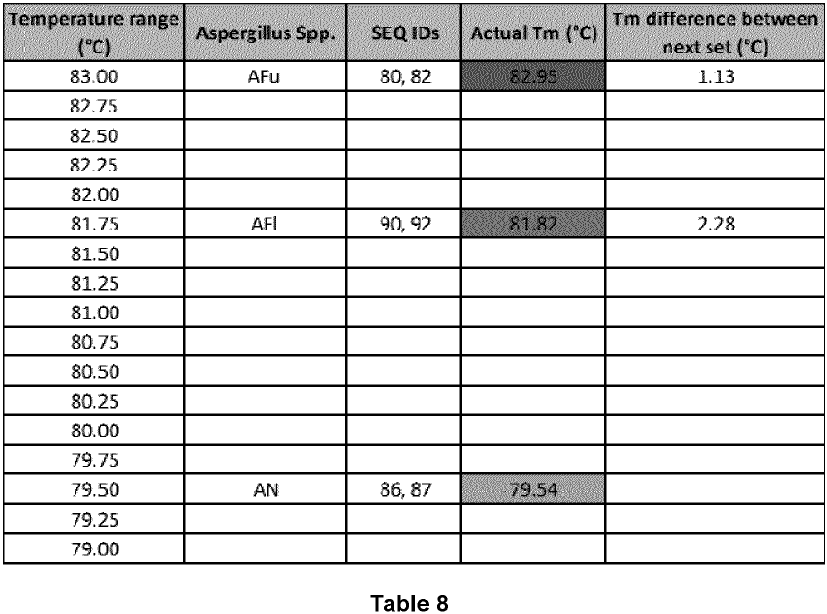

Each individual primer-probe set described herein, and shown in the accompanying Table B has been experimentally tested and validated, without any cross-reactivity of the various *ILV3* primer-probe sets to a non-target organism.

*ILV3* represents a novel gene for the identification of yeast and fungal organisms, within the context of, but not limited to, confirming their presence within blood samples of patients suspected of having bloodstream infections.

### References:

- Kan, V. L. (1993). Polymerase Chain Reaction for the Diagnosis of Candidemia. J. Infect. Dis. 168(3), 779-783.
- Khot, P. D., Ko, D. L. & Fredricks, D. N. (2009). Sequencing and Analysis of Fungal rRNA Operons for Development of Broad-Range Fungal PCR Assays. Appl. Environ. Microbiol. 75(6), 1559-1565.
- Liu, M., Healy, M. D., Dougherty, B. A., Esposito, K. M., Maurice, T. C., Mazzucco, C. E., Bruccoleri, R. E., Davison, D. B., Frosco, M., Barrett, J. F. & Wang, Y.-K. (2006). Conserved Fungal Genes as Potential Targets for Broad-Spectrum Antifungal Drug Discovery. Eukaryot. Cell. 5(4), 638-649.
- Public Health England (2015). Surveillance of candidaemia in England, Wales and Northern Ireland, 2014. Vol. 9, No. 33; 18 September 2015.
- Public Health England (2016). www.gov.uk/government/publications/candida-auris-emergence-in-england. Published 1 July 2016.
- Trick, W. E., Fridkin, S. K., Edwards, J. R., Hajjeh, R. A., Gaynes, R. P., National Nosocomial Infections Surveillance System Hospitals (2002). Secular trend of hospital-acquired candidemia among intensive care unit patients in the United States during 1989-1999. Clin. Infect. Dis. 35(5), 627-630.
- Wisplinghoff, H., Ebbers, J., Geurtz, L., Stefanik, D., Major, Y., Edmond, M. B., Wenzel, R. P., Seifert, H. (2014). Nosocomial bloodstream infections due to Candida spp. in the USA: species distribution, clinical features and antifungal susceptibilities. Int. J. Antimicrob. Agents. 43(1), 78-81.
- Van Burik JA, Myerson D, Schreckhise RW, Bowden RA (1998). Panfungal PCR assay for detection of fungal infection in human blood specimens. J Clin Microbiol. 1998 May;36(5):1169-75*.* 36(5):1169-75.

## Claims

1. A method of detecting a fungal/yeast infection in a sample, comprising:
a. performing a nucleic acid amplification reaction to amplify the ILV3 gene of fungi/yeast
b. detecting the amplification product to determine whether the sample contains a fungal/yeast infection,
wherein the sample comprises a clinical sample obtained from a human subject.

2. The method of claim 1, wherein the nucleic acid amplification reaction comprises the following components:
i. a forward and reverse primer hybridizing specifically to the ILV3 gene of *Candida* species, optionally together with a probe that hybridizes between the primer binding sites specifically to the ILV3 gene of *Candida* species; and
ii.a forward and reverse primer hybridizing specifically to the ILV3 gene of *Aspergillus* species, optionally together with a probe that hybridizes between the primer binding sites specifically to the ILV3 gene of *Aspergillus* species; and/or
iii. a forward and reverse primer hybridizing specifically to the ILV3 gene of *Cryptococcus neoformans,* optionally together with a probe that hybridizes between the primer binding sites specifically to the ILV3 gene of *Cryptococcus neoformans.*

3. The method of claim 1 or 2 wherein the nucleic acid amplification reaction amplifies the ILV3 gene of at least 3, 4, 5, 6, 7, 8, 9, 10, 11 or all 12 of the following species:
i. *Candida albicans*
ii. *Candida dubliniensis*
iii. *Candida tropicalis*
iv. *Candida parapsilosis*
v. *Candida glabrata*
vi. *Candida krusei*
vii. *Candida guilliermondii*
viii. *Candida auris*
ix. *Aspergillus fumigatus*
x. *Aspergillus niger*
xi. *Aspergillus flavus*
xii. *Cryptococcus neoformans.*

4. The method of any one of claims 1 to 3 wherein step a comprises:
i. use of a forward and reverse primer hybridizing specifically to the ILV3 gene of *Candida* species, *Aspergillus* species or *Cryptococcus neoformans;* and/or
ii. use of a probe that hybridizes specifically to the ILV3 gene of *Candida* species, *Aspergillus* species or *Cryptococcus neoformans.*

5. The method of any one of claims 1 to 4 wherein a common forward and reverse primer and/or common probe hybridises to the ILV3 gene of at least 3, 4, 5, 6, 7 and preferably all, of the following *Candida* species:
i. *Candida albicans*
ii. *Candida dubliniensis*
iii. *Candida tropicalis*
iv. *Candida parapsilosis*
v. *Candida glabrata*
vi. *Candida krusei*
vii. *Candida guilliermondii*
viii. *Candida auris;*
and/or wherein a common forward and reverse primer and/or probe hybridises to the ILV3 gene of at least 2, and preferably all 3, of the following *Aspergillus* species:
i. *Aspergillus fumigatus*
ii. *Aspergillus niger*
iii. *Aspergillus flavus*

6. The method of any one of claims 1 to 3 wherein a separate forward and reverse primer and/or probe hybridises to the ILV3 gene of each of at least 3, 4, 5, 6, 7 and preferably all, of the following *Candida* species:
i. *Candida albicans*
ii. *Candida dubliniensis*
iii. *Candida tropicalis*
iv. *Candida parapsilosis*
v. *Candida glabrata*
vi. *Candida krusei*
vii. *Candida guilliermondii*
viii. *Candida auris;*
and/or wherein a separate forward and reverse primer and/or probe hybridises to the ILV3 gene of each of at least 2, and preferably all 3, of the following *Aspergillus* species:
i. *Aspergillus fumigatus*
ii. *Aspergillus niger*
iii. *Aspergillus flavus.*

7. The method of any preceding claim wherein step b comprises distinguishing amplification products in order to identify the genus and/or species responsible for the infection.

8. The method of claim 7 wherein distinguishing comprises:
i. a melting curve analysis
ii. use of differently labelled primers and/or probes, optionally wherein
a) at least one primer and/or probe is differentially labelled according to genus to permit identification of the genus of fungus/yeast in the sample; or
b) wherein at least one primer and/or probe is differentially labelled according to species of *Candida* and/or *Aspergillus* to permit identification of the species of *Candida* and/or *Aspergillus* in the sample; or
iii. determining the size of the amplification products.

9. A collection of primer pairs for detecting a yeast/fungus infection in a sample, wherein the sample comprises a clinical sample obtained from a human subject, comprising:
A)
a. a forward and reverse primer hybridizing specifically to the ILV3 gene of the following *Candida* species
i. *Candida albicans*
ii. *Candida dubliniensis*
iii. *Candida tropicalis*
iv. *Candida parapsilosis*
*v. Candida glabrata*
vi. *Candida krusei*
vii. *Candida guilliermondii*
viii. *Candida auris*
b. a forward and reverse primer hybridizing specifically to the ILV3 gene of the following *Aspergillus* species
i. *Aspergillus fumigatus*
ii. *Aspergillus niger*
III. *Aspergillus flavus;* and
optionally a forward and reverse primer hybridizing specifically to the ILV3 gene of *Cryptococcus neoformans;* or
B)
a. a forward and reverse primer hybridizing specifically to the ILV3 gene of *Candida albicans*
b. a forward and reverse primer hybridizing specifically to the ILV3 gene of *Candida dubliniensis*
c. a forward and reverse primer hybridizing specifically to the ILV3 gene of *Candida tropicalis*
d. a forward and reverse primer hybridizing specifically to the ILV3 gene of *Candida parapsilosis*
e. a forward and reverse primer hybridizing specifically to the ILV3 gene of *Candida glabrata*
f. a forward and reverse primer hybridizing specifically to the ILV3 gene of *Candida krusei*
g. a forward and reverse primer hybridizing specifically to the ILV3 gene of *Candida* guilliermondii
h. a forward and reverse primer hybridizing specifically to the ILV3 gene of *Candida auris*
i. a forward and reverse primer hybridizing specifically to the ILV3 gene of *Aspergillus fumigatus;*
j. a forward and reverse primer hybridizing specifically to the ILV3 gene of *Aspergillus niger;*
k. a forward and reverse primer hybridizing specifically to the ILV3 gene of *Aspergillus flavus*; and optionally
l. a forward and reverse primer hybridizing specifically to the ILV3 gene of *Cryptococcus neoformans;*
optionally wherein at least one primer in each primer pair is differentially labelled compared to the other primer pairs.

10. A collection of probes for detecting a yeast/fungus infection in a sample, wherein the sample comprises a clinical sample obtained from a human subject, comprising:
A)
a. a probe that hybridizes specifically to the ILV3 gene of the following *Candida* species
i. *Candida albicans*
ii. *Candida dubliniensis*
iii. *Candida tropicalis*
iv. *Candida parapsilosis*
v. *Candida glabrata*
vi. *Candida krusei*
*vii. Candida guilliermondii*
viii. *Candida auris*
b. a probe that hybridizes specifically to the ILV3 gene of the following *Aspergillus* species
i. *Aspergillus fumigatus*
ii. *Aspergillus niger*
iii. *Aspergillus flavus; and*
optionally a probe that hybridizes specifically to the ILV3 gene of *Cryptococcus neoformans;* or
B)
a. a probe that hybridizes specifically to the ILV3 gene of *Candida albicans*
b. a probe that hybridizes specifically to the ILV3 gene of *Candida dubliniensis*
c. a probe that hybridizes specifically to the ILV3 gene of *Candida tropicalis*
d. a probe that hybridizes specifically to the ILV3 gene of *Candida parapsilosis*
e. a probe that hybridizes specifically to the ILV3 gene of *Candida glabrata*
f. a probe that hybridizes specifically to the ILV3 gene of *Candida krusei*
g. a probe that hybridizes specifically to the ILV3 gene of
*Candida guilliermondii*
h. a probe that hybridizes specifically to the ILV3 gene of *Candida auris*
i. a probe that hybridizes specifically to the ILV3 gene of *Aspergillus fumigatus*
j. a probe that hybridizes specifically to the ILV3 gene of *Aspergillus niger*
k. a probe that hybridizes specifically to the ILV3 gene of *Aspergillus flavus;* and optionally
l. a probe that hybridizes specifically to the ILV3 gene of *Cryptococcus neoformans;*
optionally wherein the collection of probes comprises at least two probes wherein each probe is differentially labelled.

11. The method of any one of claims 1 to 8, wherein the method is performed using the collection of primer pairs as defined in claim 9 and/or the collection of probes as defined in claim 10.

12. A kit for detecting a yeast/fungus infection in a sample comprising the collection of primer pairs according to claim 9 and/or the collection of probes according to claim 10; optionally wherein the kit comprises primer pairs in combination permitting detection of *Candida, Aspergillus* and *Cryptococcus neoformans;* optionally wherein the kit further comprises comprising probes permitting detection of *Candida, Aspergillus* and *Cryptococcus neoformans;* optionally wherein the kit further comprises reagents for extracting DNA from a blood sample.

13. A method of detecting and identifying a microbial infection in a sample, comprising:
a. performing a nucleic acid amplification reaction comprising the following components:
i. a forward and reverse primer hybridizing specifically to the 16S rRNA gene of Gram positive bacteria; optionally together with a probe that hybridizes between the primer binding sites specifically to the 16S rRNA gene of Gram positive bacteria
ii. a forward and reverse primer hybridizing specifically to the 16S rRNA gene of Gram negative bacteria; optionally together with a probe that hybridizes between the primer binding sites specifically to the 16S rRNA gene of Gram negative bacteria
iii. a forward and reverse primer hybridizing specifically to the ILV3 gene of at least one fungal/yeast species; optionally together with a probe that hybridizes between the primer binding sites specifically to the ILV3 gene of at least one fungal/yeast species
b. detecting and distinguishing the amplification products to determine whether the sample contains a Gram negative bacterial infection, a Gram positive bacterial infection and/or a fungal/yeast infection;
wherein the sample comprises a clinical sample obtained from a human subject; optionally wherein the amplification of the ILV3 gene is performed according to a method as defined in any one of claims 1 to 8 and/or wherein the method is performed using the collection of primer pairs as defined in claim 9 and/or wherein the method is performed using the collection of probes as defined in claim 10.

14. A kit for discriminating a microbial infection in a sample, comprising components for performing a multiplex nucleic acid amplification reaction comprising:
a. a forward and reverse primer hybridizing specifically to the 16S rRNA gene of Gram positive bacteria; optionally together with a probe that hybridizes between the primer binding sites specifically to the 16S rRNA gene of Gram positive bacteria;
b. a forward and reverse primer hybridizing specifically to the 16S rRNA gene of Gram negative bacteria; optionally together with a probe that hybridizes between the primer binding sites specifically to the 16S rRNA gene of Gram negative bacteria; and
c. a forward and reverse primer hybridizing specifically to the ILV3 gene of at least one fungal/yeast species; optionally together with a probe that hybridizes between the primer binding sites specifically to the ILV3 gene of at least one fungal/yeast species;
wherein components a, b and c each produce distinguishable amplification products thus enabling a determination of whether the sample contains a Gram negative bacterial infection, a Gram positive bacterial infection and/or a fungal/yeast infection.

15. The kit of claim 14 which comprises the collection of primer pairs as defined in claim 9 and/or collection of probes according to claim 10 and/or a kit as defined in claim 12.

16. The method of any one of claims 1 to 8 which is performed as a multiplex nucleic acid amplification reaction.

17. A method of:
a) selecting a human subject for treatment with an antifungal agent such as a fungicide; or
b) predicting responsiveness of a human subject to treatment with an antifungal agent such as a fungicide comprising performing a method according to any one of claims 1 to 8, 13 and:
i) selecting the subject for treatment with an antifungal agent such as a fungicide where an infection is detected, optionally also identified; or
ii) predicting responsiveness of the subject to treatment with an antifungal agent such as a fungicide where an infection is detected, optionally also identified.

18. An antifungal agent such as a fungicide for use in a method of treating an infection comprising administering an antifungal agent such as a fungicide to a human subject suffering from the infection, wherein:
a) the subject has been selected for treatment by performing a method according to any one of claims 1 to 8, 13; or
b) the subject displays, in a sample, a detectable ILV3 gene, and wherein the sample comprises a clinical sample obtained from a human subject.

19. The method of any one of claims 1 to 8, 11, 13, or 16, the collection of primer pairs of claim 9, the collection of probes of claim 10, or the kit of claim 12, 14 or 15, wherein the sample comprises a blood sample.

## Patentansprüche

1. Verfahren zum Nachweis einer Pilz-/Hefeinfektion in einer Probe, umfassend:
a. Durchführung einer Nukleinsäure-Amplifikationsreaktion zur Amplifikation des ILV3-Gens von Pilzen/Hefen
b. Nachweis des Amplifikationsprodukts, um festzustellen, ob die Probe eine Pilz-/Hefeinfektion enthält,
wobei die Probe eine klinische Probe umfasst, die von einem menschlichen Individuum erhalten wurde.

2. Verfahren nach Anspruch 1, wobei die Nukleinsäure-Amplifikationsreaktion die folgenden Komponenten umfasst:
i. einen Vorwärts- und einen Rückwärtsprimer, die spezifisch an das ILV3-Gen von Candida-Spezies hybridisieren, gegebenenfalls zusammen mit einer Sonde, die zwischen den Primerbindungsstellen spezifisch an das ILV3-Gen von *Candida-*Spezies hybridisiert; und
ii. einen Vorwärts- und einen Rückwärtsprimer, die spezifisch an das ILV3-Gen von *Aspergillus*-Spezies hybridisieren, gegebenenfalls zusammen mit einer Sonde, die zwischen den Primerbindungsstellen spezifisch an das ILV3-Gen von *Aspergillus*-Spezies hybridisiert; und/oder
iii. einen Vorwärts- und einen Rückwärtsprimer, die spezifisch an das ILV3-Gen von *Cryptococcus neoformans* hybridisieren, gegebenenfalls zusammen mit einer Sonde, die zwischen den Primerbindungsstellen spezifisch an das ILV3-Gen von *Cryptococcus neoformans* hybridisiert.

3. Verfahren nach Anspruch 1 oder 2, wobei die Nukleinsäure-Amplifikationsreaktion das ILV3-Gen von mindestens 3, 4, 5, 6, 7, 8, 9, 10, 11 oder allen 12 der folgenden Spezies amplifiziert:
i. *Candida albicans*
ii. *Candida dubliniensis*
iii. *Candida tropicalis*
iv. *Candida parapsilosis*
v. *Candida glabrata*
vi. *Candida krusei*
vii. *Candida guilliermondii*
viii. *Candida auris*
ix. *Aspergillus fumigatus*
x. *Aspergillus niger*
xi. *Aspergillus flavus*
xii. *Cryptococcus neoformans.*

4. Verfahren nach einem der Ansprüche 1 bis 3, wobei Schritt a umfasst:
i. Verwendung eines Vorwärts- und Rückwärtsprimers, die spezifisch an das ILV3-Gen von *Candida*-Spezies, *Aspergillus-*Spezies oder *Cryptococcus neoformans* hybridisieren; und/oder
ii. Verwendung einer Sonde, die spezifisch an das ILV3-Gen von *Candida-*Spezies, *Aspergillus-*Spezies oder *Cryptococcus neoformans* hybridisiert.

5. Verfahren nach einem der Ansprüche 1 bis 4, wobei ein gemeinsamer Vorwärts- und Rückwärtsprimer und/oder eine gemeinsame Sonde an das ILV3-Gen von mindestens 3, 4, 5, 6, 7 und vorzugsweise allen der folgenden *Candida-*Spezies hybridisiert:
i. *Candida albicans*
ii. *Candida dubliniensis*
iii. *Candida tropicalis*
iv. *Candida parapsilosis*
v. *Candida glabrata*
vi. *Candida krusei*
vii. *Candida guilliermondii*
viii. *Candida auris;*
und/oder wobei ein gemeinsamer Vorwärts- und Rückwärtsprimer und/oder eine Sonde an das ILV3-Gen von mindestens 2, vorzugsweise allen 3, der folgenden Aspergillus-Spezies hybridisiert:
i. *Aspergillus fumigatus*
ii. *Aspergillus niger*
iii. *Aspergillus flavus.*

6. Verfahren nach einem der Ansprüche 1 bis 3, wobei ein separater Vorwärts-und Rückwärtsprimer und/oder eine Sonde an das ILV3-Gen jeder der mindestens 3, 4, 5, 6, 7 und vorzugsweise aller der folgenden *Candida*-Spezies hybridisiert:
i. *Candida albicans*
ii. *Candida dubliniensis*
iii. *Candida tropicalis*
iv. *Candida parapsilosis*
v. *Candida glabrata*
vi. *Candida krusei*
vii. *Candida guilliermondi*
viii. *Candida auris;*
und/oder wobei ein separater Vorwärts- und Rückwärtsprimer und/oder eine Sonde an das ILV3-Gen von mindestens 2, und vorzugsweise allen 3, der folgenden Aspergillus-Spezies hybridisiert
i. *Aspergillus fumigatus*
ii. *Aspergillus niger*
iii. *Aspergillus flavus.*

7. Verfahren nach einem der vorhergehenden Ansprüche, wobei Schritt b das Unterscheiden von Amplifikationsprodukten umfasst, um die für die Infektion verantwortliche Gattung und/oder Spezies zu identifizieren.

8. Verfahren nach Anspruch 7, wobei das Unterscheiden umfasst:
i. eine Schmelzkurvenanalyse
ii. Verwendung von unterschiedlich markierten Primern und/oder Sonden, wobei gegebenenfalls
a) mindestens ein Primer und/oder eine Sonde je nach Gattung unterschiedlich markiert ist, um die Identifizierung der Pilz-/Hefengattung in der Probe zu ermöglichen; oder
b) wobei mindestens ein Primer und/oder eine Sonde je nach Spezies von *Candida* und/oder *Aspergillus* unterschiedlich markiert ist, um die Identifizierung der Spezies von *Candida* und/oder *Aspergillus* in der Probe zu ermöglichen; oder
iii. Bestimmung der Größe der Amplifikationsprodukte.

9. Sammlung von Primerpaaren zum Nachweis einer Hefe-/Pilzinfektion in einer Probe, wobei die Probe eine klinische Probe umfasst, die von einem menschlichen Individuum erhalten wurde, umfassend:
A)
a. einen Vorwärts- und einen Rückwärtsprimer, die spezifisch an das ILV3-Gen der folgenden Candida-Spezies hybridisieren
i. *Candida albicans*
ii. *Candida dubliniensis*
iii. *Candida tropicalis*
iv. *Candida parapsilosis*
v. *Candida glabrata*
vi. *Candida krusei*
vii. *Candida guilliermondi*
viii. *Candida auris*
b. einen Vorwärts- und einen Rückwärtsprimer, die spezifisch an das ILV3-Gen der folgenden *Aspergillus*-Spezies hybridisieren
i. *Aspergillus fumigatus*
ii. *Aspergillus niger*
iii. *Aspergillus flavus;* und
gegebenenfalls einen Vorwärts- und einen Rückwärtsprimer, die spezifisch an das ILV3-Gen von *Cryptococcus neoformans* hybridisieren; oder
B)
a. einen Vorwärts- und einen Rückwärtsprimer, die spezifisch an das ILV3-Gen von *Candida albicans* hybridisieren
b. einen Vorwärts- und einen Rückwärtsprimer, die spezifisch an das ILV3-Gen von *Candida dubliniensis* hybridisieren
c. einen Vorwärts- und einen Rückwärtsprimer, die spezifisch an das ILV3-Gen von *Candida tropicalis* hybridisieren
d. einen Vorwärts- und einen Rückwärtsprimer, die spezifisch an das ILV3-Gen von *Candida parapsilosis* hybridisieren
e. einen Vorwärts- und einen Rückwärtsprimer, die spezifisch an das ILV3-Gen von *Candida glabrata* hybridisieren
f. einen Vorwärts- und einen Rückwärtsprimer, die spezifisch an das ILV3-Gen von *Candida krusei* hybridisieren
g. einen Vorwärts- und einen Rückwärtsprimer, die spezifisch an das ILV3-Gen von *Candida guilliermondi* hybridisieren
h. einen Vorwärts- und einen Rückwärtsprimer, die spezifisch an das ILV3-Gen von *Candida auris* hybridisieren
i. einen Vorwärts- und einen Rückwärtsprimer, die spezifisch an das ILV3-Gen von *Aspergillus fumigatus* hybridisieren
j. einen Vorwärts- und einen Rückwärtsprimer, die spezifisch an das ILV3-Gen von *Aspergillus niger* hybridisieren
k. einen Vorwärts- und einen Rückwärtsprimer, die spezifisch an das ILV3-Gen von *Aspergillus flavus* hybridisieren; und gegebenenfalls
l. einen Vorwärts- und einen Rückwärtsprimer, die spezifisch an das ILV3-Gen von *Cryptococcus neoformans* hybridisieren;
wobei gegebenenfalls mindestens ein Primer in jedem Primerpaar im Vergleich zu den anderen Primerpaaren unterschiedlich markiert ist.

10. Sammlung von Sonden zum Nachweis einer Hefe-/Pilzinfektion in einer Probe, wobei die Probe eine klinische Probe umfasst, die von einem menschlichen Individuum erhalten wurde, umfassend:
A)
a. eine Sonde, die spezifisch an das ILV3-Gen der folgenden Candida-Spezies hybridisiert
i. *Candida albicans*
ii. *Candida dubliniensis*
iii. *Candida tropicalis*
iv. *Candida parapsilosis*
v. *Candida glabrata*
vi. *Candida krusei*
vii. *Candida guilliermondi*
viii. *Candida auris*
b. eine Sonde, die spezifisch an das ILV3-Gen der folgenden *Aspergillus*-Spezies hybridisiert
i. *Aspergillus fumigatus*
ii. *Aspergillus niger*
iii. *Aspergillus flavus;* und
gegebenenfalls eine Sonde, die spezifisch an das ILV3-Gen von *Cryptococcus neoformans* hybridisiert; oder
B)
a. eine Sonde, die spezifisch an das ILV3-Gen von *Candida albicans* hybridisiert
b. eine Sonde, die spezifisch an das ILV3-Gen von *Candida dubliniensis* hybridisiert
c. eine Sonde, die spezifisch an das ILV3-Gen von *Candida tropicalis* hybridisiert
d. eine Sonde, die spezifisch an das ILV3-Gen von *Candida parapsilosis* hybridisiert
e. eine Sonde, die spezifisch an das ILV3-Gen von *Candida glabrata* hybridisiert
f. eine Sonde, die spezifisch an das ILV3-Gen von *Candida krusei* hybridisiert
g. eine Sonde, die spezifisch an das ILV3-Gen von *Candida guilliermondi* hybridisiert
h. eine Sonde, die spezifisch an das ILV3-Gen von *Candida auris* hybridisiert
i. eine Sonde, die spezifisch an das ILV3-Gen von *Aspergillus fumigatus* hybridisiert
j. eine Sonde, die spezifisch an das ILV3-Gen von *Aspergillus niger* hybridisiert
k. eine Sonde, die spezifisch an das ILV3-Gen von *Aspergillus flavus* hybridisiert; und gegebenenfalls
l. eine Sonde, die spezifisch an das ILV3-Gen von *Cryptococcus neoformans* hybridisiert;
wobei die Sammlung an Sonden gegebenenfalls wenigstens zwei Sonden umfasst, wobei jede Sonde unterschiedlich markiert ist.

11. Verfahren nach einem der Ansprüche 1 bis 8, wobei das Verfahren unter Verwendung der in Anspruch 9 definierten Sammlung von Primerpaaren und/oder der in Anspruch 10 definierten Sammlung von Sonden durchgeführt wird.

12. Kit zum Nachweis einer Hefe-/Pilzinfektion in einer Probe, umfassend die Sammlung von Primerpaaren nach Anspruch 9 und/oder die Sammlung von Sonden nach Anspruch 10; wobei das Kit gegebenenfalls Primerpaare in Kombination umfasst, die den Nachweis von *Candida, Aspergillus* und *Cryptococcus neoformans* erlauben; wobei das Kit gegebenenfalls ferner Sonden umfasst, die den Nachweis von *Candida, Aspergillus* und *Cryptococcus neoformans* erlauben; wobei das Kit gegebenenfalls ferner Reagenzien zur Extraktion von DNA aus einer Blutprobe umfasst.

13. Verfahren zum Nachweis und zur Identifizierung einer mikrobiellen Infektion in einer Probe, umfassend:
a. Durchführung einer Nukleinsäure-Amplifikationsreaktion, umfassend die folgenden Komponenten:
i. einen Vorwärts- und einen Rückwärtsprimer, die spezifisch an das 16S rRNA-Gen von Gram-positiven Bakterien hybridisieren; gegebenenfalls zusammen mit einer Sonde, die zwischen den Primerbindungsstellen spezifisch an das 16S rRNA-Gen von Gram-positiven Bakterien hybridisiert
ii. einen Vorwärts- und einen Rückwärtsprimer, die spezifisch an das 16S rRNA-Gen von Gram-negativen Bakterien hybridisieren; gegebenenfalls zusammen mit einer Sonde, die zwischen den Primerbindungsstellen spezifisch an das 16S rRNA-Gen von Gram-negativen Bakterien hybridisiert
iii. einen Vorwärts- und einen Rückwärtsprimer, die spezifisch an das ILV3-Gen von mindestens einer Pilz-/Hefe-Spezies hybridisieren; gegebenenfalls zusammen mit einer Sonde, die zwischen den Primerbindungsstellen spezifisch an das ILV3-Gen von mindestens einer Pilz-/Hefe-Spezies hybridisiert
b. Nachweis und Unterscheidung der Amplifikationsprodukte, um zu bestimmen, ob die Probe eine Gram-negative bakterielle Infektion, eine Gram-positive bakterielle Infektion und/oder eine Pilz-/Hefe-Infektion enthält;
wobei die Probe eine klinische Probe umfasst, die von einem menschlichen Individuum erhalten wurde; wobei gegebenenfalls die Amplifikation des ILV3-Gens gemäß einem Verfahren, wie in einem der Ansprüche 1 bis 8 definiert, durchgeführt wird und/oder wobei das Verfahren unter Verwendung der Sammlung von Primerpaaren, wie in Anspruch 9 definiert, durchgeführt wird und/oder wobei das Verfahren unter Verwendung der Sammlung von Sonden, wie in Anspruch 10 definiert, durchgeführt wird.

14. Kit zur Unterscheidung einer mikrobiellen Infektion in einer Probe, umfassend Komponenten zur Durchführung einer Multiplex-Nukleinsäure-Amplifikationsreaktion, umfassend:
a. einen Vorwärts- und einen Rückwärtsprimer, die spezifisch an das 16S rRNA-Gen von Gram-positiven Bakterien hybridisieren; gegebenenfalls zusammen mit einer Sonde, die zwischen den Primerbindungsstellen spezifisch an das 16S rRNA-Gen von Gram-positiven Bakterien hybridisiert
b. einen Vorwärts- und einen Rückwärtsprimer, die spezifisch an das 16S rRNA-Gen von Gram-negativen Bakterien hybridisieren; gegebenenfalls zusammen mit einer Sonde, die zwischen den Primerbindungsstellen spezifisch an das 16S rRNA-Gen von Gram-negativen Bakterien hybridisiert
c. einen Vorwärts- und einen Rückwärtsprimer, die spezifisch an das ILV3-Gen von mindestens einer Pilz-/Hefe-Spezies hybridisieren; gegebenenfalls zusammen mit einer Sonde, die zwischen den Primerbindungsstellen spezifisch an das ILV3-Gen von mindestens einer Pilz-/Hefe-Spezies hybridisiert
wobei die Komponenten a, b und c jeweils unterscheidbare Amplifikationsprodukte erzeugen, wodurch eine Bestimmung ermöglicht wird, ob die Probe eine Gram-negative bakterielle Infektion, eine Gram-positive bakterielle Infektion und/oder eine Pilz-/Hefe-Infektion enthält.

15. Kit nach Anspruch 14, welches die Sammlung von Primerpaaren nach Anspruch 9 und/oder die Sammlung von Sonden nach Anspruch 10 und/oder ein Kit nach Anspruch 12 umfasst.

16. Verfahren nach einem der Ansprüche 1 bis 8, welches als eine Multiplex-Nukleinsäure-Amplifikationsreaktion durchgeführt wird.

17. Verfahren zur:
a) Auswahl eines menschlichen Individuums für die Behandlung mit einem antifungalen Mittel, wie z.B. einem Fungizid; oder
b) Vorhersage des Ansprechens eines menschlichen Individuums auf die Behandlung mit einem antifungalen Mittel, wie einem Fungizid umfassend die Durchführung eines Verfahrens nach einem der Ansprüche 1 bis 8 und:
i) Auswählen des Individuums für die Behandlung mit einem antifungalen Mittel, wie z. B. einem Fungizid, wenn eine Infektion nachgewiesen, gegebenenfalls auch identifiziert wird; oder
ii) Vorhersage des Ansprechens des Individuums auf die Behandlung mit einem antifungalen Mittel wie einem Fungizid, wenn eine Infektion festgestellt, gegebenenfalls auch identifiziert wird.

18. Antifungales Mittel, wie ein Fungizid, zur Verwendung in einem Verfahren zur Behandlung einer Infektion, das die Verabreichung eines antifungalen Mittels, wie eines Fungizids, an ein menschliches Individuum, das an der Infektion leidet, umfasst, wobei:
a) das Individuum zur Behandlung ausgewählt wurde, indem ein Verfahren nach einem der Ansprüche 1 bis 8, 13 durchgeführt wurde; oder
b) das Individuum in einer Probe ein nachweisbares ILV3-Gen aufweist, und wobei die Probe eine klinische Probe umfasst, die von einem menschlichen Individuum erhalten wurde.

19. Verfahren nach einem der Ansprüche 1 bis 8, 11, 13 oder 16, die Sammlung von Primerpaaren nach Anspruch 9, die Sammlung von Sonden nach Anspruch 10 oder das Kit nach Anspruch 12, 14 oder 15, wobei die Probe eine Blutprobe umfasst.

## Revendications

1. Procédé de détection d'une infection fongique/à levures dans un échantillon, comprenant les étapes consistant à :
a. effectuer une réaction d'amplification d'acide nucléique pour amplifier le gène ILV3 de champignons/levures
b. détecter le produit d'amplification pour déterminer si l'échantillon contient une infection fongique/à levures,
dans lequel l'échantillon comprend un échantillon clinique obtenu à partir d'un sujet humain.

2. Procédé de la revendication 1, dans lequel la réaction d'amplification d'acide nucléique comprend les composants suivants :
i. une amorce directe et inverse s'hybridant spécifiquement au gène ILV3 des espèces de *Candida,* facultativement conjointement avec une sonde qui s'hybride entre les sites de liaison d'amorce spécifiquement au gène ILV3 des espèces de *Candida* ; et
ii. une amorce directe et inverse s'hybridant spécifiquement au gène ILV3 des espèces *d'Aspergillus,* facultativement conjointement avec une sonde qui s'hybride entre les sites de liaison d'amorce spécifiquement au gène ILV3 des espèces d'*Aspergillus* ; et/ou
iii. une amorce directe et inverse s'hybridant spécifiquement au gène ILV3 de *Cryptococcus neoformans,* facultativement conjointement avec une sonde qui s'hybride entre les sites de liaison d'amorce spécifiquement au gène ILV3 de *Cryptococcus neoformans.*

3. Procédé de la revendication 1 ou 2, dans lequel la réaction d'amplification d'acide nucléique amplifie le gène ILV3 d'au moins 3, 4, 5, 6, 7, 8, 9, 10, 11 espèces ou de l'ensemble des 12 espèces suivantes :
i. *Candida albicans*
ii. *Candida dubliniensis*
iii. *Candida tropicalis*
iv. *Candida parapsilosis*
v. *Candida glabrata*
vi. *Candida krusei*
vii. *Candida guilliermondii*
viii. *Candida auris*
ix. *Aspergillus fumigatus*
x. *Aspergillus niger*
xi. *Aspergillus flavus*
xii. *Cryptococcus neoformans.*

4. Procédé de l'une quelconque des revendications 1 à 3, dans lequel l'étape a comprend :
i. l'utilisation d'une amorce directe et inverse s'hybridant spécifiquement au gène ILV3 des espèces de *Candida,* des espèces *d'Aspergillus* ou de *Cryptococcus neoformans ;* et/ou
ii. l'utilisation d'une sonde qui s'hybride spécifiquement au gène ILV3 des espèces de *Candida,* des espèces *d'Aspergillus* ou de *Cryptococcus neoformans.*

5. Procédé de l'une quelconque des revendications 1 à 4, dans lequel une amorce directe et inverse commune et/ou une sonde commune s'hybrident au gène ILV3 d'au moins 3, 4, 5, 6, 7 espèces et de préférence de toutes les espèces de *Candida* suivantes :
i. *Candida albicans*
ii. *Candida dubliniensis*
iii. *Candida tropicalis*
iv. *Candida parapsilosis*
v. *Candida glabrata*
vi. *Candida krusei*
vii. *Candida guilliermondii*
viii. *Candida auris ;*
et/ou dans lequel une amorce directe et inverse et/ou une sonde commune(s) s'hybrident au gène ILV3 d'au moins 2 espèces, et de préférence de l'ensemble des 3 espèces *d'Aspergillus* suivantes :
i. *Aspergillus fumigatus*
ii. *Aspergillus niger*
iii. *Aspergillus flavus.*

6. Procédé de l'une quelconque des revendications 1 à 3, dans lequel une amorce directe et inverse et/ou une sonde distincte(s) s'hybrident au gène ILV3 de chacune d'au moins 3, 4, 5, 6, 7 espèces et de préférence de toutes les espèces de *Candida* suivantes :
i. *Candida albicans*
ii. *Candida dubliniensis*
iii. *Candida tropicalis*
iv. *Candida parapsilosis*
v. *Candida glabrata*
vi. *Candida krusei*
vii. *Candida guilliermondii*
viii. *Candida auris ;*
et/ou dans lequel une amorce directe et inverse et/ou une sonde distincte(s) s'hybrident au gène ILV3 de chacune d'au moins 2 espèces, et de préférence de l'ensemble des 3 espèces *d'Aspergillus* suivantes :
i. *Aspergillus fumigatus*
ii. *Aspergillus niger*
iii. *Aspergillus flavus.*

7. Procédé de l'une des revendications précédentes, dans lequel l'étape b comprend la distinction des produits d'amplification afin d'identifier le genre et/ou l'espèce responsables de l'infection.

8. Procédé de la revendication 7, dans lequel la distinction comprend :
i. une analyse de courbe de fusion
ii. l'utilisation d'amorces et/ou de sondes marquées différemment, facultativement où
a) au moins une amorce et/ou une sonde sont marquées de manière différentielle selon le genre pour permettre l'identification du genre de champignon/levure dans l'échantillon ; ou
b) dans lequel au moins une amorce et/ou une sonde sont marquées de manière différentielle selon les espèces de *Candida* et/ou *d'Aspergillus* pour permettre l'identification des espèces de *Candida* et/ou *d'Aspergillus* dans l'échantillon ; ou
iii. la détermination de la taille des produits d'amplification.

9. Collection de paires d'amorces pour détecter une infection à levures/fongique dans un échantillon, où l'échantillon comprend un échantillon clinique obtenu à partir d'un sujet humain, comprenant :
A)
a. une amorce directe et inverse s'hybridant spécifiquement au gène ILV3 des espèces de *Candida* suivantes
i. *Candida albicans*
ii. *Candida dubliniensis*
iii. *Candida tropicalis*
iv. *Candida parapsilosis*
v. *Candida glabrata*
vi. *Candida krusei*
vii. *Candida guilliermondii*
viii. *Candida auris*
b. une amorce directe et inverse s'hybridant spécifiquement au gène ILV3 des espèces d'*Aspergillus* suivante
i. *Aspergillus fumigatus*
ii. *Aspergillus niger*
iii. *Aspergillus flavus* ; et
facultativement une amorce directe et inverse s'hybridant spécifiquement au gène ILV3 de *Cryptococcus neoformans* ; ou
B)
a. une amorce directe et inverse s'hybridant spécifiquement au gène ILV3 de *Candida albicans*
b. une amorce directe et inverse s'hybridant spécifiquement au gène ILV3 de *Candida dubliniensis*
c. une amorce directe et inverse s'hybridant spécifiquement au gène ILV3 de *Candida tropicalis*
d. une amorce directe et inverse s'hybridant spécifiquement au gène ILV3 de *Candida parapsilosis*
e. une amorce directe et inverse s'hybridant spécifiquement au gène ILV3 de *Candida glabrata*
f. une amorce directe et inverse s'hybridant spécifiquement au gène ILV3 de *Candida krusei*
g. une amorce directe et inverse s'hybridant spécifiquement au gène ILV3 de *Candida guilliermondii*
h. une amorce directe et inverse s'hybridant spécifiquement au gène ILV3 de *Candida auris*
i. une amorce directe et inverse s'hybridant spécifiquement au gène ILV3 d'*Aspergillus fumigatus ;*
j. une amorce directe et inverse s'hybridant spécifiquement au gène ILV3 d'*Aspergillus niger ;*
k. une amorce directe et inverse s'hybridant spécifiquement au gène ILV3 d'*Aspergillus flavus* ; et facultativement
l. une amorce directe et inverse s'hybridant spécifiquement au gène ILV3 de *Cryptococcus neoformans ;*
facultativement dans laquelle au moins une amorce dans chaque paire d'amorces est marquée de manière différentielle par rapport aux autres paires d'amorces.

10. Collection de sondes pour détecter une infection à levures/fongique dans un échantillon, où l'échantillon comprend un échantillon clinique obtenu à partir d'un sujet humain, comprenant :
A)
a. une sonde qui s'hybride spécifiquement au gène ILV3 des espèces de *Candida* suivantes
i. *Candida albicans*
ii. *Candida dubliniensis*
iii. *Candida tropicalis*
iv. *Candida parapsilosis*
v. *Candida glabrata*
vi. *Candida krusei*
vii. *Candida guilliermondii*
viii. *Candida auris*
b. une sonde qui s'hybride spécifiquement au gène ILV3 des espèces d'*Aspergillus* suivantes
i. *Aspergillus fumigatus*
ii. *Aspergillus niger*
iii. *Aspergillus flavus* ; et
facultativement une sonde qui s'hybride spécifiquement au gène ILV3 de *Cryptococcus neoformans* ; ou
B)
a. une sonde qui s'hybride spécifiquement au gène ILV3 de *Candida albicans*
b. une sonde qui s'hybride spécifiquement au gène ILV3 de *Candida dubliniensis*
c. une sonde qui s'hybride spécifiquement au gène ILV3 de *Candida tropicalis*
d. une sonde qui s'hybride spécifiquement au gène ILV3 de *Candida parapsilosis*
e. une sonde qui s'hybride spécifiquement au gène ILV3 de *Candida glabrata*
f. une sonde qui s'hybride spécifiquement au gène ILV3 de *Candida krusei*
g. une sonde qui s'hybride spécifiquement au gène ILV3 de *Candida guilliermondii*
h. une sonde qui s'hybride spécifiquement au gène ILV3 de *Candida auris*
i. une sonde qui s'hybride spécifiquement au gène ILV3 *d'Aspergillus fumigatus*
j. une sonde qui s'hybride spécifiquement au gène ILV3 d'*Aspergillus niger*
k. une sonde qui s'hybride spécifiquement au gène ILV3 d'*Aspergillus flavus* ; et facultativement
l. une sonde qui s'hybride spécifiquement au gène ILV3 de *Cryptococcus neoformans ;*
facultativement, où la collection de sondes comprend au moins deux sondes, où chaque sonde est marquée de manière différentielle.

11. Procédé de l'une quelconque des revendications 1 à 8, où le procédé est effectué en utilisant la collection de paires d'amorces telle que définie dans la revendication 9 et/ou la collection de sondes telle que définie dans la revendication 10.

12. kit de détection d'une infection à levures/fongique dans un échantillon comprenant la collection de paires d'amorces selon la revendication 9 et/ou la collection de sondes selon la revendication 10 ; facultativement où le kit comprend des paires d'amorces en combinaison permettant la détection de *Candida,* d'*Aspergillus* et de *Cryptococcus neoformans ;* facultativement où le kit comprend en outre des sondes permettant la détection de *Candida,* d'*Aspergillus* et de *Cryptococcus neoformans* ; facultativement où le kit comprend en outre des réactifs pour extraire de l'ADN d'un échantillon de sang.

13. Procédé de détection et d'identification d'une infection microbienne dans un échantillon, comprenant les étapes consistant à :
a. effectuer une réaction d'amplification d'acide nucléique comprenant les composants suivants :
i. une amorce directe et inverse s'hybridant spécifiquement au gène d'ARNr 16S de bactéries Gram positives ; facultativement conjointement avec une sonde qui s'hybride entre les sites de liaison d'amorce spécifiquement au gène d'ARNr 16S des bactéries Gram positives
ii. une amorce directe et inverse s'hybridant spécifiquement au gène d'ARNr 16S de bactéries Gram négatives ; facultativement conjointement avec une sonde qui s'hybride entre les sites de liaison d'amorce spécifiquement au gène d'ARNr 16S des bactéries Gram négatives
iii. une amorce directe et inverse s'hybridant spécifiquement au gène ILV3 d'au moins une espèce de champignon/levure ; facultativement conjointement avec une sonde qui s'hybride entre les sites de liaison d'amorce spécifiquement au gène ILV3 d'au moins une espèce de champignon/levure
b. détecter et distinguer les produits d'amplification pour déterminer si l'échantillon contient une infection bactérienne à Gram négatif, une infection bactérienne à Gram positif et/ou une infection fongique/à levures ;
dans lequel l'échantillon comprend un échantillon clinique obtenu à partir d'un sujet humain ; facultativement dans lequel l'amplification du gène ILV3 est effectuée selon un procédé tel que défini dans l'une quelconque des revendications 1 à 8 et/ou où le procédé est effectué en utilisant la collection de paires d'amorces telle que définie dans la revendication 9 et/ou où le procédé est effectué en utilisant la collection de sondes telle que définie dans la revendication 10.

14. Kit de distinction d'une infection microbienne dans un échantillon, comprenant des composants pour effectuer une réaction d'amplification d'acide nucléique multiplex comprenant :
a. une amorce directe et inverse s'hybridant spécifiquement au gène d'ARNr 16S de bactéries Gram positives ; facultativement conjointement avec une sonde qui s'hybride entre les sites de liaison d'amorce spécifiquement au gène d'ARNr 16S des bactéries Gram positives ;
b. une amorce directe et inverse s'hybridant spécifiquement au gène d'ARNr 16S de bactéries Gram négatives ; facultativement conjointement avec une sonde qui s'hybride entre les sites de liaison d'amorce spécifiquement au gène d'ARNr 16S des bactéries Gram négatives ; et
c. une amorce directe et inverse s'hybridant spécifiquement au gène ILV3 d'au moins une espèce de champignon/levure ; facultativement conjointement avec une sonde qui s'hybride entre les sites de liaison d'amorce spécifiquement au gène ILV3 d'au moins une espèce de champignon/levure ;
dans lequel les composants a, b et c produisent chacun des produits d'amplification pouvant être distingués permettant ainsi de déterminer si l'échantillon contient une infection bactérienne à Gram négatif, une infection bactérienne à Gram positif et/ou une infection fongique/à levures.

15. Kit de la revendication 14 qui comprend la collection de paires d'amorces telle que définie dans la revendication 9 et/ou la collection de sondes selon la revendication 10 et/ou un kit tel que défini dans la revendication 12.

16. Procédé de l'une quelconque des revendications 1 à 8, qui est effectué en tant que réaction d'amplification d'acide nucléique multiplex.

17. Procédé consistant en :
a) la sélection d'un sujet humain pour un traitement avec un agent antifongique tel qu'un fongicide ; ou
b) la prédiction de la faculté de réponse d'un sujet humain à un traitement avec un agent antifongique tel qu'un fongicide
comprenant l'étape consistant à effectuer un procédé selon l'une quelconque des revendications 1 à 8, et :
i) la sélection du sujet pour un traitement avec un agent antifongique tel qu'un fongicide où une infection est détectée, facultativement, également identifiée ; ou
ii) la prédiction de la faculté de réponse du sujet au traitement avec un agent antifongique tel qu'un fongicide où une infection est détectée, facultativement, également identifiée.

18. Agent antifongique tel qu'un fongicide pour une utilisation dans un procédé de traitement d'une infection comprenant l'administration d'un agent antifongique tel qu'un fongicide à un sujet humain souffrant de l'infection, où :
a) le sujet a été sélectionné pour le traitement en effectuant un procédé selon l'une quelconque des revendications 1 à 8, 13 ; ou
b) le sujet présente, dans un échantillon, un gène ILV3 détectable, et où l'échantillon comprend un échantillon clinique obtenu à partir d'un sujet humain.

19. Procédé de l'une quelconque des revendications 1 à 8, 11, 13 ou 16, collection de paires d'amorces de la revendication 9, collection de sondes de la revendication 10, ou kit de la revendication 12, 14 ou 15, où l'échantillon comprend un échantillon de sang.


### SEQUENCE LISTING

<110> Momentum Bioscience Ltd
<120> DETECTION AND DELINEATION OF MICROORGANISMS
<130> MPS/P142470WO00
<150> GB1705932.0
   <151> 2017-04-12
<150> GB1711949.6
   <151> 2017-07-25
<160> 146
<170> PatentIn version 3.5
<210> 1
   <211> 20
   <212> DNA
   <213> Artificial Sequence
<220>
   <223> Probe or primer sequence
<400> 1
   gaaggyccaa arggtggwcc 20
<210> 2
   <211> 23
   <212> DNA
   <213> Artificial Sequence
<220>
   <223> Probe or primer sequence
<400> 2
   gawccaccmg araatctrcc rtc 23
<210> 3
   <211> 26
   <212> DNA
   <213> Artificial Sequence
<220>
   <223> Probe or primer sequence
<400> 3
   gawggyttca acatttcygg catacc 26
<210> 4
   <211> 18
   <212> DNA
   <213> Artificial Sequence
<220>
   <223> Probe or primer sequence
<400> 4
   tcccttgttg gccgattt 18
<210> 5
   <211> 22
   <212> DNA
   <213> Artificial Sequence
<220>
   <223> Probe or primer sequence
<400> 5
   taccgtcgat aatgccttct tt 22
<210> 6
   <211> 22
   <212> DNA
   <213> Artificial Sequence
<220>
   <223> Probe or primer sequence
<400> 6
   aatcttgcag agggtgtctt ag 22
<210> 7
   <211> 19
   <212> DNA
   <213> Artificial Sequence
<220>
   <223> Probe or primer sequence
<400> 7
   gggtcgcctg tgacaatag 19
<210> 8
   <211> 21
   <212> DNA
   <213> Artificial Sequence
<220>
   <223> Probe or primer sequence
<400> 8
   cagtaaatag ggctggcttg a 21
<210> 9
   <211> 22
   <212> DNA
   <213> Artificial Sequence
<220>
   <223> Probe or primer sequence
<400> 9
   ccttctgtac cgttggtgat ac 22
<210> 10
   <211> 20
   <212> DNA
   <213> Artificial Sequence
<220>
   <223> Probe or primer sequence
<400> 10
   tgcctcgtcg tttgacatta 20
<210> 11
   <211> 21
   <212> DNA
   <213> Artificial Sequence
<220>
   <223> Probe or primer sequence
<400> 11
   gtacttaccg gatggcttga a 21
<210> 12
   <211> 20
   <212> DNA
   <213> Artificial Sequence
<220>
   <223> Probe or primer sequence
<400> 12
   cattgccact ggtggttcta 20
<210> 13
   <211> 22
   <212> DNA
   <213> Artificial Sequence
<220>
   <223> Probe or primer sequence
<400> 13
   gatggcttga agtcggctaa ta 22
<210> 14
   <211> 20
   <212> DNA
   <213> Artificial Sequence
<220>
   <223> Probe or primer sequence
<400> 14
   ccaggttctg ctgttggtaa 20
<210> 15
   <211> 22
   <212> DNA
   <213> Artificial Sequence
<220>
   <223> Probe or primer sequence
<400> 15
   ccaaagcagt gatgaaggaa tg 22
<210> 16
   <211> 23
   <212> DNA
   <213> Artificial Sequence
<220>
   <223> Probe or primer sequence
<400> 16
   gttctgctgt tggtaaaatc act 23
<210> 17
   <211> 22
   <212> DNA
   <213> Artificial Sequence
<220>
   <223> Probe or primer sequence
<400> 17
   caaagcagtg atgaaggaat gt 22
<210> 18
   <211> 21
   <212> DNA
   <213> Artificial Sequence
<220>
   <223> Probe or primer sequence
<400> 18
   gccatgggaa gacacaatag a 21
<210> 19
   <211> 23
   <212> DNA
   <213> Artificial Sequence
<220>
   <223> Probe or primer sequence
<400> 19
   tcttgtcggc aatagctgga tta 23
<210> 20
   <211> 19
   <212> DNA
   <213> Artificial Sequence
<220>
   <223> Probe or primer sequence
<400> 20
   gtggtggaag caagagtaa 19
<210> 21
   <211> 22
   <212> DNA
   <213> Artificial Sequence
<220>
   <223> Probe or primer sequence
<400> 21
   gcttctcttc caaagtgatt tg 22
<210> 22
   <211> 23
   <212> DNA
   <213> Artificial Sequence
<220>
   <223> Probe or primer sequence
<400> 22
   ttcaagccat ctggtaagta tgt 23
<210> 23
   <211> 22
   <212> DNA
   <213> Artificial Sequence
<220>
   <223> Probe or primer sequence
<400> 23
   cagttaaggc gtcaccgtat aa 22
<210> 24
   <211> 20
   <212> DNA
   <213> Artificial Sequence
<220>
   <223> Probe or primer sequence
<400> 24
   taaggccgaa tggaaacctc 20
<210> 25
   <211> 20
   <212> DNA
   <213> Artificial Sequence
<220>
   <223> Probe or primer sequence
<400> 25
   acaaccttgg gaggcattag 20
<210> 26
   <211> 22
   <212> DNA
   <213> Artificial Sequence
<220>
   <223> Probe or primer sequence
<400> 26
   gcctctcagg ctatgttgta tg 22
<210> 27
   <211> 20
   <212> DNA
   <213> Artificial Sequence
<220>
   <223> Probe or primer sequence
<400> 27
   accagaccac caacaagaac 20
<210> 28
   <211> 22
   <212> DNA
   <213> Artificial Sequence
<220>
   <223> Probe or primer sequence
<400> 28
   gacggtatct cgatgggtac ta 22
<210> 29
   <211> 21
   <212> DNA
   <213> Artificial Sequence
<220>
   <223> Probe or primer sequence
<400> 29
   atcgtagtgt tgtgccatca t 21
<210> 30
   <211> 22
   <212> DNA
   <213> Artificial Sequence
<220>
   <223> Probe or primer sequence
<400> 30
   atgggttacg gcttaggtaa ag 22
<210> 31
   <211> 22
   <212> DNA
   <213> Artificial Sequence
<220>
   <223> Probe or primer sequence
<400> 31
   tctggaacaa tatggccgat ta 22
<210> 32
   <211> 22
   <212> DNA
   <213> Artificial Sequence
<220>
   <223> Probe or primer sequence
<400> 32
   ggtggtatgt acactgccaa ta 22
<210> 33
   <211> 20
   <212> DNA
   <213> Artificial Sequence
<220>
   <223> Probe or primer sequence
<400> 33
   ccgaaactgc tggagatgat 20
<210> 34
   <211> 21
   <212> DNA
   <213> Artificial Sequence
<220>
   <223> Probe or primer sequence
<400> 34
   tggtttcaag gacgaggatt t 21
<210> 35
   <211> 23
   <212> DNA
   <213> Artificial Sequence
<220>
   <223> Probe or primer sequence
<400> 35
   cagaatctgc acatgcctta ttt 23
<210> 36
   <211> 20
   <212> DNA
   <213> Artificial Sequence
<220>
   <223> Probe or primer sequence
<400> 36
   gcggttctca cggtttctta 20
<210> 37
   <211> 22
   <212> DNA
   <213> Artificial Sequence
<220>
   <223> Probe or primer sequence
<400> 37
   ggcaagttct tcttcggata ca 22
<210> 38
   <211> 22
   <212> DNA
   <213> Artificial Sequence
<220>
   <223> Probe or primer sequence
<400> 38
   ggaagaggcc agagttgaaa ta 22
<210> 39
   <211> 20
   <212> DNA
   <213> Artificial Sequence
<220>
   <223> Probe or primer sequence
<400> 39
   cctttggagg catcagagac 20
<210> 40
   <211> 22
   <212> DNA
   <213> Artificial Sequence
<220>
   <223> Probe or primer sequence
<400> 40
   gttcacgacg gagatgagat tg 22
<210> 41
   <211> 22
   <212> DNA
   <213> Artificial Sequence
<220>
   <223> Probe or primer sequence
<400> 41
   ggagaccaca gcttctttct tt 22
<210> 42
   <211> 20
   <212> DNA
   <213> Artificial Sequence
<220>
   <223> Probe or primer sequence
<400> 42
   tcgatcgtga cccaggataa 20
<210> 43
   <211> 22
   <212> DNA
   <213> Artificial Sequence
<220>
   <223> Probe or primer sequence
<400> 43
   gaccaccata cacttccaac tc 22
<210> 44
   <211> 22
   <212> DNA
   <213> Artificial Sequence
<220>
   <223> Probe or primer sequence
<400> 44
   gtgacccagg ataagtctca ag 22
<210> 45
   <211> 20
   <212> DNA
   <213> Artificial Sequence
<220>
   <223> Probe or primer sequence
<400> 45
   ggtttcctga ccaccataca 20
<210> 46
   <211> 22
   <212> DNA
   <213> Artificial Sequence
<220>
   <223> Probe or primer sequence
<400> 46
   caaggctgga ttgaaaggaa tg 22
<210> 47
   <211> 21
   <212> DNA
   <213> Artificial Sequence
<220>
   <223> Probe or primer sequence
<400> 47
   acatctgggc catcactaaa g 21
<210> 48
   <211> 23
   <212> DNA
   <213> Artificial Sequence
<220>
   <223> Probe or primer sequence
<400> 48
   ctcctctgta ggcgttgaaa tta 23
<210> 49
   <211> 22
   <212> DNA
   <213> Artificial Sequence
<220>
   <223> Probe or primer sequence
<400> 49
   caagtcagcc atcacgtact ta 22
<210> 50
   <211> 22
   <212> DNA
   <213> Artificial Sequence
<220>
   <223> Probe or primer sequence
<400> 50
   caccggtaag gaaggaacat ac 22
<210> 51
   <211> 22
   <212> DNA
   <213> Artificial Sequence
<220>
   <223> Probe or primer sequence
<400> 51
   ccagcctgta aagcagtgat aa 22
<210> 52
   <211> 22
   <212> DNA
   <213> Artificial Sequence
<220>
   <223> Probe or primer sequence
<400> 52
   gccaagatgt tgttggaaga ag 22
<210> 53
   <211> 22
   <212> DNA
   <213> Artificial Sequence
<220>
   <223> Probe or primer sequence
<400> 53
   ggcatttgct caagttctct tt 22
<210> 54
   <211> 20
   <212> DNA
   <213> Artificial Sequence
<220>
   <223> Probe or primer sequence
<400> 54
   aacatgcctg gtgtgcttat 20
<210> 55
   <211> 22
   <212> DNA
   <213> Artificial Sequence
<220>
   <223> Probe or primer sequence
<400> 55
   ggcataatgg taccaccgta aa 22
<210> 56
   <211> 24
   <212> DNA
   <213> Artificial Sequence
<220>
   <223> Probe or primer sequence
<400> 56
   actggtaaga cactcaaaga gaac 24
<210> 57
   <211> 22
   <212> DNA
   <213> Artificial Sequence
<220>
   <223> Probe or primer sequence
<400> 57
   ggtttcaatg ggttggacaa ag 22
<210> 58
   <211> 24
   <212> DNA
   <213> Artificial Sequence
<220>
   <223> Probe or primer sequence
<400> 58
   gaggaggact ttatcactgc ttta 24
<210> 59
   <211> 22
   <212> DNA
   <213> Artificial Sequence
<220>
   <223> Probe or primer sequence
<400> 59
   tctgataaca cacacggtct tt 22
<210> 60
   <211> 21
   <212> DNA
   <213> Artificial Sequence
<220>
   <223> Probe or primer sequence
<400> 60
   attgaacaag cactcctcga t 21
<210> 61
   <211> 22
   <212> DNA
   <213> Artificial Sequence
<220>
   <223> Probe or primer sequence
<400> 61
   ccttaaaccc agtagcgtac aa 22
<210> 62
   <211> 22
   <212> DNA
   <213> Artificial Sequence
<220>
   <223> Probe or primer sequence
<400> 62
   gcacttctaa cagacggaag at 22
<210> 63
   <211> 21
   <212> DNA
   <213> Artificial Sequence
<220>
   <223> Probe or primer sequence
<400> 63
   tgggacaatg tgaccaatca a 21
<210> 64
   <211> 22
   <212> DNA
   <213> Artificial Sequence
<220>
   <223> Probe or primer sequence
<400> 64
   ggtaaagcca tcagacactt ga 22
<210> 65
   <211> 23
   <212> DNA
   <213> Artificial Sequence
<220>
   <223> Probe or primer sequence
<400> 65
   acctccagtg gcaatgatat aag 23
<210> 66
   <211> 23
   <212> DNA
   <213> Artificial Sequence
<220>
   <223> Probe or primer sequence
<400> 66
   catggtttac ggtggtacca tta 23
<210> 67
   <211> 22
   <212> DNA
   <213> Artificial Sequence
<220>
   <223> Probe or primer sequence
<400> 67
   ccgtatgatt ggaaagcaga ga 22
<210> 68
   <211> 22
   <212> DNA
   <213> Artificial Sequence
<220>
   <223> Probe or primer sequence
<400> 68
   caacagaatt gacttgctcg tg 22
<210> 69
   <211> 23
   <212> DNA
   <213> Artificial Sequence
<220>
   <223> Probe or primer sequence
<400> 69
   cgcaaagtac ctctcttgta tct 23
<210> 70
   <211> 15
   <212> DNA
   <213> Artificial Sequence
<220>
   <223> Probe or primer sequence
<400> 70
   scagggtgct tcsca 15
<210> 71
   <211> 16
   <212> DNA
   <213> Artificial Sequence
<220>
   <223> Probe or primer sequence
<400> 71
   tsgcrtcgta ccactg 16
<210> 72
   <211> 25
   <212> DNA
   <213> Artificial Sequence
<220>
   <223> Probe or primer sequence
<400> 72
   cagtatgggt acaaagggwa tgmga 25
<210> 73
   <211> 20
   <212> DNA
   <213> Artificial Sequence
<220>
   <223> Probe or primer sequence
<400> 73
   ggtacsaagg gwatgcgata 20
<210> 74
   <211> 20
   <212> DNA
   <213> Artificial Sequence
<220>
   <223> Probe or primer sequence
<400> 74
   ctgatgttcg crtcrtacca 20
<210> 75
   <211> 23
   <212> DNA
   <213> Artificial Sequence
<220>
   <223> Probe or primer sequence
<400> 75
   tcyatcgara ccgtyatggg tgg 23
<210> 76
   <211> 22
   <212> DNA
   <213> Artificial Sequence
<220>
   <223> Probe or primer sequence
<400> 76
   gcgaagaaac ggctttgaat aa 22
<210> 77
   <211> 23
   <212> DNA
   <213> Artificial Sequence
<220>
   <223> Probe or primer sequence
<400> 77
   ttgtaccaga cagacgaaat acc 23
<210> 78
   <211> 24
   <212> DNA
   <213> Artificial Sequence
<220>
   <223> Probe or primer sequence
<400> 78
   acctgcgcct tgttcatatc ctcc 24
<210> 79
   <211> 21
   <212> DNA
   <213> Artificial Sequence
<220>
   <223> Probe or primer sequence
<400> 79
   gcctcacaga ggaggatatg a 21
<210> 80
   <211> 20
   <212> DNA
   <213> Artificial Sequence
<220>
   <223> Probe or primer sequence
<400> 80
   atccagcagg tgcatgttac 20
<210> 81
   <211> 24
   <212> DNA
   <213> Artificial Sequence
<220>
   <223> Probe or primer sequence
<400> 81
   ttcgtctgtc tggtacaacg gcaa 24
<210> 82
   <211> 23
   <212> DNA
   <213> Artificial Sequence
<220>
   <223> Probe or primer sequence
<400> 82
   aggcctcaca gaggaggata tga 23
<210> 83
   <211> 21
   <212> DNA
   <213> Artificial Sequence
<220>
   <223> Probe or primer sequence
<400> 83
   gtaccaaggg aatgcgatac t 21
<210> 84
   <211> 21
   <212> DNA
   <213> Artificial Sequence
<220>
   <223> Probe or primer sequence
<400> 84
   gatactgatg ttcgcgtcgt a 21
<210> 85
   <211> 23
   <212> DNA
   <213> Artificial Sequence
<220>
   <223> Probe or primer sequence
<400> 85
   tccatcgaaa ccgtcatggg tgg 23
<210> 86
   <211> 20
   <212> DNA
   <213> Artificial Sequence
<220>
   <223> Probe or primer sequence
<400> 86
   ctggtgtgcc accgatatta 20
<210> 87
   <211> 21
   <212> DNA
   <213> Artificial Sequence
<220>
   <223> Probe or primer sequence
<400> 87
   attgctgact ctgtcggtat c 21
<210> 88
   <211> 24
   <212> DNA
   <213> Artificial Sequence
<220>
   <223> Probe or primer sequence
<400> 88
   agacaccgtt cctggctgat ttga 24
<210> 89
   <211> 23
   <212> DNA
   <213> Artificial Sequence
<220>
   <223> Probe or primer sequence
<400> 89
   gctataccaa acggaggaga tac 23
<210> 90
   <211> 19
   <212> DNA
   <213> Artificial Sequence
<220>
   <223> Probe or primer sequence
<400> 90
   cttcccaggc catgcttta 19
<210> 91
   <211> 24
   <212> DNA
   <213> Artificial Sequence
<220>
   <223> Probe or primer sequence
<400> 91
   accgaggagg acatgaacaa agct 24
<210> 92
   <211> 22
   <212> DNA
   <213> Artificial Sequence
<220>
   <223> Probe or primer sequence
<400> 92
   ctataccaaa cggaggagat ac 22
<210> 93
   <211> 22
   <212> DNA
   <213> Artificial Sequence
<220>
   <223> Probe or primer sequence
<400> 93
   gttcaagacg gcgatgttat tc 22
<210> 94
   <211> 20
   <212> DNA
   <213> Artificial Sequence
<220>
   <223> Probe or primer sequence
<400> 94
   gcaacccagt tctcctttct 20
<210> 95
   <211> 24
   <212> DNA
   <213> Artificial Sequence
<220>
   <223> Probe or primer sequence
<400> 95
   atgccgttgc gaacactttg tctg 24
<210> 96
   <211> 22
   <212> DNA
   <213> Artificial Sequence
<220>
   <223> Probe or primer sequence
<400> 96
   ggtatgcctg aaatgctcaa ac 22
<210> 97
   <211> 21
   <212> DNA
   <213> Artificial Sequence
<220>
   <223> Probe or primer sequence
<400> 97
   ccatgagaac ctccgctaaa t 21
<210> 98
   <211> 24
   <212> DNA
   <213> Artificial Sequence
<220>
   <223> Probe or primer sequence
<400> 98
   agtttgatca tgggcgctgg tcta 24
<210> 99
   <211> 22
   <212> DNA
   <213> Artificial Sequence
<220>
   <223> Probe or primer sequence
<400> 99
   acactttgtc tgtggacgta tc 22
<210> 100
   <211> 20
   <212> DNA
   <213> Artificial Sequence
<220>
   <223> Probe or primer sequence
<400> 100
   gtaacagctc gggcgtattt 20
<210> 101
   <211> 25
   <212> DNA
   <213> Artificial Sequence
<220>
   <223> Probe or primer sequence
<400> 101
   tcaaggtcac tcacggaaca ttgct 25
<210> 102
   <211> 20
   <212> DNA
   <213> Artificial Sequence
<220>
   <223> Probe or primer sequence
<400> 102
   cgtcgaaagc ggttctatca 20
<210> 103
   <211> 22
   <212> DNA
   <213> Artificial Sequence
<220>
   <223> Probe or primer sequence
<400> 103
   ggtttgagca tttcaggcat ac 22
<210> 104
   <211> 24
   <212> DNA
   <213> Artificial Sequence
<220>
   <223> Probe or primer sequence
<400> 104
   tgttgtcttg aggtaccttg gccc 24
<210> 105
   <211> 23
   <212> DNA
   <213> Artificial Sequence
<220>
   <223> Probe or primer sequence
<400> 105
   tcgacccagt atgatggttt atg 23
<210> 106
   <211> 21
   <212> DNA
   <213> Artificial Sequence
<220>
   <223> Probe or primer sequence
<400> 106
   tttggccttc ttggaggtat c 21
<210> 107
   <211> 24
   <212> DNA
   <213> Artificial Sequence
<220>
   <223> Probe or primer sequence
<400> 107
   cggagaggtg ctcgacattg tgtc 24
<210> 108
   <211> 16
   <212> DNA
   <213> Artificial Sequence
<220>
   <223> Probe or primer sequence
<400> 108
   tacgggaggc agcagt 16
<210> 109
   <211> 16
   <212> DNA
   <213> Artificial Sequence
<220>
   <223> Probe or primer sequence
<400> 109
   tattaccgcg gctgct 16
<210> 110
   <211> 28
   <212> DNA
   <213> Artificial Sequence
<220>
   <223> Probe or primer sequence
<400> 110
   ccgcagaata agcaccggct aactccgt 28
<210> 111
   <211> 30
   <212> DNA
   <213> Artificial Sequence
<220>
   <223> Probe or primer sequence
<400> 111
   cctaaccaga aagccacggc taactacgtg 30
<210> 112
   <211> 19
   <212> DNA
   <213> Artificial Sequence
<220>
   <223> Probe or primer sequence
<400> 112
   caacgcgaag aaccttacc 19
<210> 113
   <211> 18
   <212> DNA
   <213> Artificial Sequence
<220>
   <223> Probe or primer sequence
<400> 113
   acgtcatccc caccttcc 18
<210> 114
   <211> 21
   <212> DNA
   <213> Artificial Sequence
<220>
   <223> Probe or primer sequence
<400> 114
   acgacaacca tgcaccacct g 21
<210> 115
   <211> 20
   <212> DNA
   <213> Artificial Sequence
<220>
   <223> Probe or primer sequence
<400> 115
   acgacagcca tgcagcacct 20
<210> 116
   <211> 24
   <212> DNA
   <213> Artificial Sequence
<220>
   <223> Probe or primer sequence
<400> 116
   cggcggtaca cctgctgtta tgaa 24
<210> 117
   <211> 28
   <212> DNA
   <213> Artificial Sequence
<220>
   <223> Probe or primer seqeuence
<400> 117
   tgcttgattg tggatgtagc aaatgtcc 28
<210> 118
   <211> 24
   <212> DNA
   <213> Artificial Sequence
<220>
   <223> Probe or primer sequence
<400> 118
   aattgcatgg ctttcaagcc agcc 24
<210> 119
   <211> 24
   <212> DNA
   <213> Artificial Sequence
<220>
   <223> Probe or primer sequence
<400> 119
   attagccgac ttcaagccat ccgg 24
<210> 120
   <211> 26
   <212> DNA
   <213> Artificial Sequence
<220>
   <223> Probe or primer sequence
<400> 120
   tgcctcgtcg tttgacatta ccatca 26
<210> 121
   <211> 30
   <212> DNA
   <213> Artificial Sequence
<220>
   <223> Probe or primer sequence
<400> 121
   aggtacttac ttcaagggta aagctagagt 30
<210> 122
   <211> 25
   <212> DNA
   <213> Artificial Sequence
<220>
   <223> Probe or primer sequence
<400> 122
   tactatcttg ccagggtctc ccaca 25
<210> 123
   <211> 24
   <212> DNA
   <213> Artificial Sequence
<220>
   <223> Probe or primer sequence
<400> 123
   tcctgctatt gctgacaaga ttga 24
<210> 124
   <211> 24
   <212> DNA
   <213> Artificial Sequence
<220>
   <223> Probe or primer sequence
<400> 124
   aggtcggtgg tactcaaagt gtca 24
<210> 125
   <211> 26
   <212> DNA
   <213> Artificial Sequence
<220>
   <223> Probe or primer sequence
<400> 125
   tgctaagtta gtctctaatg cctccc 26
<210> 126
   <211> 24
   <212> DNA
   <213> Artificial Sequence
<220>
   <223> Probe or primer sequence
<400> 126
   tcagcaaagc gcaagttggt gttg 24
<210> 127
   <211> 30
   <212> DNA
   <213> Artificial Sequence
<220>
   <223> Probe or primer sequence
<400> 127
   agagaaatta tcgcagactc tttcgagact 30
<210> 128
   <211> 24
   <212> DNA
   <213> Artificial Sequence
<220>
   <223> Probe or primer sequence
<400> 128
   attctctggc ggttctcacg gttt 24
<210> 129
   <211> 24
   <212> DNA
   <213> Artificial Sequence
<220>
   <223> Probe or primer sequence
<400> 129
   acccaagact tctgaggctg aagc 24
<210> 130
   <211> 24
   <212> DNA
   <213> Artificial Sequence
<220>
   <223> Probe or primer sequence
<400> 130
   tggttcttgc tggtggtctg gaaa 24
<210> 131
   <211> 24
   <212> DNA
   <213> Artificial Sequence
<220>
   <223> Probe or primer sequence
<400> 131
   agaagctttc gaaggcggtc caat 24
<210> 132
   <211> 26
   <212> DNA
   <213> Artificial Sequence
<220>
   <223> Probe or primer sequence
<400> 132
   cctctcttat atttcaactc tggcct 26
<210> 133
   <211> 26
   <212> DNA
   <213> Artificial Sequence
<220>
   <223> Probe or primer sequence
<400> 133
   tgcttgtctc ggaagaaatt ctcgct 26
<210> 134
   <211> 24
   <212> DNA
   <213> Artificial Sequence
<220>
   <223> Probe or primer sequence
<400> 134
   tgcttcgcaa gccatgttgt atgc 24
<210> 135
   <211> 27
   <212> DNA
   <213> Artificial Sequence
<220>
   <223> Probe or primer sequence
<400> 135
   accgttggtg ataccatcag aaactcc 27
<210> 136
   <211> 24
   <212> DNA
   <213> Artificial Sequence
<220>
   <223> Probe or primer sequence
<400> 136
   tctgacagca ctccattgtt ggct 24
<210> 137
   <211> 24
   <212> DNA
   <213> Artificial Sequence
<220>
   <223> Probe or primer sequence
<400> 137
   aaaggctaga gtgtttgacg ccga 24
<210> 138
   <211> 25
   <212> DNA
   <213> Artificial Sequence
<220>
   <223> Probe or primer sequence
<400> 138
   tgcttgatgg tgaccagatg actgt 25
<210> 139
   <211> 25
   <212> DNA
   <213> Artificial Sequence
<220>
   <223> Probe or primer sequence
<400> 139
   aaggtctgtt gtgtctaccc atggc 25
<210> 140
   <211> 22
   <212> DNA
   <213> Artificial Sequence
<220>
   <223> Probe or primer sequence
<400> 140
   atatcctggc cctcaggcaa gc 22
<210> 141
   <211> 24
   <212> DNA
   <213> Artificial Sequence
<220>
   <223> Probe or primer sequence
<400> 141
   tcgcccttct tgatttctcc agcc 24
<210> 142
   <211> 22
   <212> DNA
   <213> Artificial Sequence
<220>
   <223> Probe or primer sequence
<400> 142
   tgtcaccgag gatgtgtcgc ag 22
<210> 143
   <211> 23
   <212> DNA
   <213> Artificial Sequence
<220>
   <223> Probe or primer sequence
<400> 143
   tttctggtgg atcccacggt ttc 23
<210> 144
   <211> 27
   <212> DNA
   <213> Artificial Sequence
<220>
   <223> Probe or primer sequence
<400> 144
   cccgtgacat catgaccaag aaatcgt 27
<210> 145
   <211> 24
   <212> DNA
   <213> Artificial Sequence
<220>
   <223> Probe or primer sequence
<400> 145
   tggccatggg tagacacaac agac 24
<210> 146
   <211> 24
   <212> DNA
   <213> Artificial Sequence
<220>
   <223> Probe or primer sequence
<400> 146
   aagaaagaag tggctgcctc ctga 24